# EUROPÄISCHE PATENTSCHRIFT

(11) **EP 1 282 610 B1**
(45) Veröffentlichungstag und Bekanntmachung des Hinweises auf die Patenterteilung: **25.07.2007**
(21) Anmeldenummer: 01929561.7
(22) Anmeldetag: 12.04.2001
(51) Int. Cl.: C07D 265/36, C07D 265/34, A61K 31/538

(54) **BENZOXAZIN- UND BENZOTHIAZIN-DERIVATE UND DEREN VERWENDUNG IN ARZNEIMITTELN**
BENZOXAZINE AND BENZOTHIAZINE DERIVATIVES AND THE USE THEREOF IN MEDICAMENTS
DERIVES DE BENZOXAZINE ET DE BENZOTHIAZINE ET LEUR UTILISATION DANS DES MEDICAMENTS

(30) Priorität: 19.04.2000 DE 10020667
(43) Veröffentlichungstag der Anmeldung: 12.02.2003
(73) Patentinhaber: Bayer Schering Pharma Aktiengesellschaft, 13353 Berlin (DE)
(72) Erfinder: REHWINKEL, Hartmut, 12051 Berlin (DE); HÖLSCHER, Peter, 10559 Berlin (DE); JAROCH, Stefan, 10625 Berlin (DE); SÜLZLE, Detlev, 10589 Berlin (DE); HILLMANN, Margrit, 13437 Berlin (DE); BURTON, Gerardine, Anne, 13591 Berlin (DE); MCDONALD, Fiona, Mcdougall, 14055 Berlin (DE)
(74) Vertreter: Pohlman, Sandra M.
(86) Internationale Anmeldenummer: PCT/EP2001/004281
(87) Internationale Veröffentlichungsnummer: WO 2001/081323

(56) Entgegenhaltungen:
- WO-A-00/17173
- WO-A-01/14347
- WO-A-98/50372
- WO-A-99/12915
- M SUDOH: "Identification of a novel Inhibitor specific to the Fungal chitin synthase" J BIOL CHEM, Bd. 275, Nr. 42, 20. August 2000 (2000-08-20), Seiten 32901-32905, XP000999470

## Beschreibung

Die Erfindung betrifft Benzoxazin- und Benzothiazin-Derivate, das Verfahren zu deren Herstellung und deren Verwendung in Arzneimitteln.

In menschlichen Zellen existieren mindestens 3 Formen von Stickstoffmonoxid-Synthasen, die Arginin in Stickstoffmonoxid (NO) und Citrullin überführen. Es wurden zwei konstitutive NO-Synthasen (NOS) identifiziert, die als Calcium / Calmodulin abhängige Enzyme im Gehirn (ncNOS oder NOS 1) bzw. im Endothel (ecNOS oder NOS 3) vorhanden sind. Eine weitere Isoform ist die induzierbare NOS (iNOS oder NOS 2), die ein praktisch Ca⁺⁺ unabhängiges Enzym ist und nach Aktivierung unterschiedlicher Zellen durch Endotoxin oder andere Stoffe induziert wird.

NOS-Inhibitoren und insbesondere selektive Inhibitoren der NOS 1, NOS 2 oder NOS 3 sind daher zur Therapie unterschiedlicher Erkrankungen geeignet, die durch pathologische Konzentrationen von NO in Zellen hervorgerufen oder verschlimmert werden. Eine Reihe von Reviews informiert über Wirkung und Inhibitoren von NO-Synthasen. Genannt seien beispielsweise: Drugs 1, 321 (1998), oder Current Pharmac. Design 3, 447 (1997),

Als NOS-Inhibitoren sind unterschiedliche Verbindungen bekannt. Beispielsweise werden Argininderivate, Aminopyridine, cyclische Amidinderivate, Phenylimidazole und andere beschrieben. Aus WO 98/50372 und WO 99/12915 ist bekannt, daß 3-Amino-2H-1,4-benzoxazine oder-benzothiazine potent und selektiv Stickstoffmonoxid Synthasen hemmen.

Es wurde nun gefunden, daß die erfindungsgemäß substituierten Heterocyclen gegenüber bekannten Verbindungen Vorteile besitzen und besser als Arzneimittel eingesetzt werden können.

Die Erfindung betrifft die Verbindungen der Formel I, deren tautomere und isomere Formen und Salze 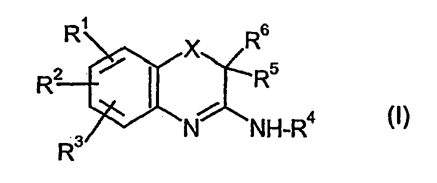 worin
- X: O oder S,
- R¹: -(CHR⁹)ₙ-NR⁷-B,
- R²: Wasserstoff oder
- R¹ und R²: gemeinsam mit zwei benachbarten Kohlenstoffatomen einen 5-; 6-, 7- oder 8- gliedrigen Ring bilden, der monocyclisch oder bicyclisch, gesättigt oder ungesättigt ist und bei dem 1 oder 2 CH₂-Gruppen durch Sauerstoff oder Carbonyl ersetzt sein können und der mit -(CHR⁹)ᵣ-NR⁷-B substituiert ist und mit C₁₋₄-Alkyl substituiert sein kann,
- R³: Wasserstoff, Halogen, NO₂, Cyano, CF₃, -OCF₃. -S-R⁹, -O-R⁹, C₃₋₇Cycloalkyl, -NR⁹-C(=NR¹⁰)-R¹¹, -NH-CS-NR¹²R¹³, NH-CO-NR¹²R¹³, -SO₂NR¹²R¹³, -CO-NR¹²R¹³, -CO-R¹⁴, NR¹⁵R¹⁶, C₆₋₁₀Aryl, das gegebenenfalls mit Halogen, Cyano, C₁₋₄-Alkyl, -S-R⁹ oder -O-R⁹ substituiert ist,
5-oder 6gliedriges Heteroaryl mit 1 - 4 Sauerstoff-, Schwefel- oder Stickstoffatomen
C₁₋₆-Alkyl, das gegebenenfalls mit Halogen, -OR⁹, -SR9, -NR¹²R¹³, =NR¹², =NOC₁₋₆-Alkyl, =N-NHAryl, Phenyl, C₃₋₇-Cycloalkyl oder 5- oder 6gliedrigem Heteroaryl substituiert ist,
C₂₋₆-Alkenyl, das gegebenenfalls mit Halogen, CONH₂, C≡N oder Phenyl substituiert ist,
C₂₋₆-Alkinyl, das gegebenenfalls mit Halogen, CONH₂, C≡N oder Phenyl substituiert ist,
- R⁴: Wasserstoff oder Acyl,
- R⁵ und R⁶: unabhängig voneinander Wasserstoff, C₃₋₇-Cycloalkyl, Phenyl, C₁₋₆-Alkyl, C₂₋₆-Alkenyl- oder C₂₋₆-Alkynylreste, die jeweils substituiert sein können mit Halogen, OH, O-C₁₋₆-Alkyl, SH, S-C₁₋₆-Alkyl, NR¹⁵R¹⁶, 5- oder 6-gliedriges Hereroaryl mit 1 - 3 N-, O- oder S-Atomen, Phenyl oder C₃₋₇-Cycloalkyl,
- R⁷: Wasserstoff, C₁₋₆-Alkyl. das mit Phenyl substituiert sein kann, COOC₁₋₆-Alkyl oder COC₁₋₆-Alkyl,
- R⁸: Wasserstoff,
- A: geradkettiges oder verzweigtes -C₁₋₆-Alkylen, geradkettiges oder verzweigtes -C₂₋₆-Alkenylen,
- B: halogeniertes C₁₋₈-Alkyl, das mit C₆₋₁₀-Aryl substituiert sein kann, wobei der Arylrest mit Halogen, Cyano, OH, C₁₋₄-Alkoxy, C₁₋₄-Alkyl, CF₃ und -OCF₃ substituiert sein kann, C₃₋₈Alkinyl, das mit C₆₋₁₀-Aryl substituiert sein kann, -(CH₂)ₚ-C₆₋₁₀-Aryl, das mit -OCF₃ substituiert ist, oder C₃₋₇-Cycloalkyl, das mit CF₃ substituiert ist,
- n: und r 0, 1 bis 6,
- p: 0 bis 6,
- R⁹ und R¹⁰: Wasserstoff oder C₁₋₆-Alkyl,
- R¹¹: C₁₋₆-Alkyl, -NH₂, -NH-CH₃, -NH-CN, gegebenenfalls mit Halogen, C₁₋₄-Alkyl oder CF₃ substituiertes C₆₋₁₀-Aryl oder gegebenenfalls mit Halogen, C₁₋₄-Alkyl oder CF₃ substituiertes 5- oder 6gliedriges Heteroaryl mit 1 bis 4 Stickstoff-, Schwefel- oder Sauerstoffatomen,
- R¹² und R¹³: Wasserstoff, C₁₋₆-Alkyl, gegebenenfalls mit Halogen oder C₁₋₄-Alkyl substituiertes Phenyl, gegebenenfalls mit Halogen oder C₁₋₄-Alkyl substituiertes Benzyl oder C₃₋₇-Cycloalkyl,
- R¹⁴: Wasserstoff, Hydroxy, C₁₋₆-Alkoxy, Phenyl, gegebenenfalls mit CO₂H, CO₂C₁₋₆-Alkyl, Hydroxy, C₁₋₄-Alkoxy, Halogen, NR¹⁵R¹⁶, CONR¹²R¹³ oder Phenyl substituiertes C₁₋₆-Alkyl oder gegebenenfalls mit Phenyl, Cyano, CONR¹²R¹³ oder CO₂C₁₋₄-Alkyl substituiertes C₂₋₆-Alkenyl,
- R¹⁵: und R¹⁶ Wasserstoff, C₁₋₆-Alkyl, gegebenenfalls mit Halogen oder C₁₋₄-Alkyl substituiertes Phenyl oder gegebenenfalls mit Halogen oder C₁₋₄-Alkyl substituiertes Benzyl oder
- R¹⁵,: R¹⁶ gemeinsam mit dem Stickstoffatom einen gesättigten 5-, 6- oder 7gliedrigen Ring bilden, der ein weiteres Stickstoff-, Sauerstoff- oder Schwefelatom enthalten und substituiert sein kann mit C₁₋₄-Alkyl- oder einem gegebenenfalls mit Halogen substituierten Phenyl-, Benzyl- oder Benzoylrest
bedeuten.

Die Verbindungen der Formel I können als Tautomere, Stereoisomere oder geometrische Isomere vorliegen. Die Erfindung umfaßt E- und Z-Isomere, S- und R-Enantiomere, Diastereomere, Racemate und Gemische derselben einschließlich der tautomeren Verbindungen der Formel Ia und Ib

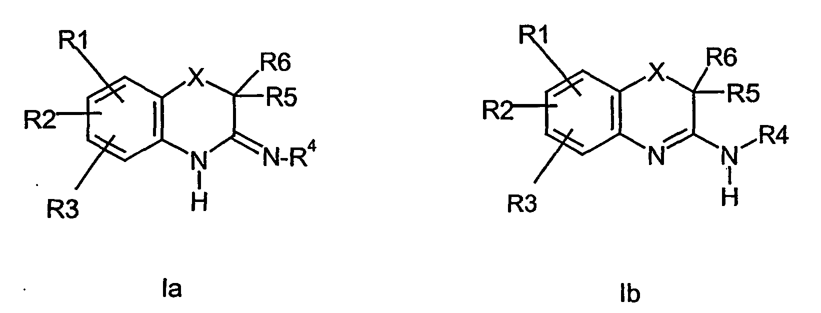

Die physiologisch verträglichen Salze können mit anorganischen und organischen Säuren gebildet werden, wie beispielsweise Oxalsäure, Milchsäure, Zitronensäure, Fumarsäure, Essigsäure, Maleinsäure, Weinsäure, Phosphorsäure, HCl, HBr, Schwefelsäure, p-Toluolsulfonsäure, Methansulfonsäure u.a.

Zur Salzbildung von Säuregruppen sind auch die anorganischen oder organischen Basen geeignet, die zur Bildung physiologisch verträglicher Salze bekannt sind wie beispielsweise Alkalihydroxide, wie Natrium- und Kaliumhydroxid, Erdalkalihydroxide wie Calciumhydroxid, Ammoniak, Amine wie Ethanolamin, Diethanolamin, Triethanolamin, N-Methylglucamin, Tris-(hydroxymethyl)-methylamin usw.

Alkyl bedeutet jeweils eine geradkettige oder verzweigte Alkylgruppe wie z.B. Methyl, Ethyl, Propyl, Isopropyl, n-Butyl, sek. Butyl, tert. Butyl, n-Pentyl, sek. Pentyl, tert. Pentyl, Neopentyl, n-Hexyl., sek. Hexyl, Heptyl, Octyl.

Ist der Alkylrest mit Halogen substituiert, so kann er ein- bis mehrfach halogeniert und perhalogeniert sein, beispielsweise genannt seien Fluormethyl, 2-Fluorethyl, 1-Fluor-prop-2-yl, 4,4,4,-Trifluorbutyl., 4,4,4-Trifluorbut-2-yl, 2,2,3,3,3-Pentafluorpropyl, 3-Fluor-3-phenyl-prop-2-yl, 3-(Trifluormethyl)-cyclohexyl, 2-(Trifluormethyl)-cyclohexyl, 2,2,2-Trifluorethyl, 2,2,3,3,4,4,4-Heptafluorbutyl, 3,3,2,2-Tetrafluor-but-1-yl, 3,3-Difluor-but-1-yl.

Alkenyl- und Alkynyl-Substituenten sind jeweils geradkettig oder verzweigt und haben insbesondere eine Doppel- oder Dreifachbindung. Beispielsweise seien die folgenden Reste genannt: Vinyl, 2-Propenyl, 1-Propenyl, 2-Butenyl, 1-Butenyl, 3-Butenyl, 2-Methyl-2-propenyl, Ethinyl, 1-Propinyl, 2-Propinyl, 1-Butinyl, 2-Butinyl, 2-Pentenyl, 4-Hexenyl.

Unter Cycloalkyl ist jeweils Cyclopropyl, Cyclobutyl, Cyclopentyl, Cyclohexyl oder Cycloheptyl zu verstehen. Als Bicyclus seien beispielsweise Bicycloheptan und Bicyclooctan genannt.

Halogen bedeutet jeweils Fluor, Chlor, Brom oder Jod.

Unter Aryl ist jeweils Naphthyl oder insbesondere Phenyl zu verstehen, das gegebenenfalls ein- bis dreifach gleich oder verschieden substituiert ist.

Als Heteroarylreste, die über das Heteroatom oder ein Kohlenstoffatom gebunden sein können, seien beispielsweise die folgenden 5- und 6-Ringheteroaromaten genannt:
Imidazol, Indol, Isooxazol, Isothiazol, Furan, Oxadiazol, Oxazol, Pyrazin, Pyridazin, Pyrimidin, Pyridin, Pyrazol, Pyrrol, Tetrazol, Thiazol, Triazol, Thiophen, Thiadiazol, Benzimidazol, Benzofuran, Benzoxazol, Isochinolin, Chinolin.

Im Falle einer Substitution des Heteroarylrestes kann dieser ein- bis dreifach gleich oder verschieden substituiert sein.

Als bevorzugte Ausführungsform für R¹¹ in der Bedeutung Heteroaryl ist Thienyl zu betrachten.

Als gesättigte Heterocyclen seien beispielsweise Piperidin, Pyrrolidin, Morpholin, Thiomorpholin, Hexahydroazepin und Piperazin genannt. Der Heterocyclus kann 1 - 3fach gleich oder verschieden substituiert sein mit C₁₋₄-Alkyl oder einem gegebenenfalls mit Halogen substituierten Phenyl-, Benzyl- oder Benzoylrest. Beispielsweise seien genannt: N-Methyl-piperazin, 2,6-Dimethylmorpholin, Phenylpiperazin oder 4-(4-Fluorbenzoyl)-piperidin.

Für die Substituenten R⁵ und R⁶ in Position 2 des Oxazins oder Thiazins ist einfache Substitution bevorzugt, wobei der Substituent R⁵ insbesondere C₁₋₆-Alkyl bedeutet und R⁶ Wasserstoff ist.

Bilden R¹ und R² gemeinsam mit zwei benachbarten Kohlenstoffatomen einen Ring, so kann dieser in Position 5, 6 oder 7, 8 oder insbesondere 6, 7 des Benzoxazins bzw. Benzothiazins stehen und hat die Formel 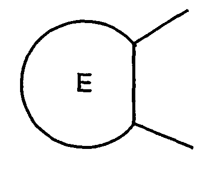 worin
E einen 3 - 8gliedrigen Ring bedeutet, der 1 - 2fach mit -(CHR⁹)ᵣ-NR⁷-A-NR⁸-B oder -(CHR⁹)ᵣ-NR⁷-B und gegebenenfalls 1 - 2fach mit C₁₋₄-Alkyl substituiert ist und bei dem 1 oder 2 CH₂-Gruppen durch Sauerstoff, Carbonyl oder dessen Derivate ersetzt sein können, und der mit Benzol anelliert sein kann, wie beispielsweise Indan, und als Bicyclus vorliegen kann, wie beispielsweise Bicyclooctan.

Als Strukturen von E seien beispielsweise genannt:

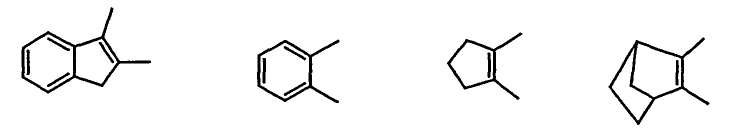

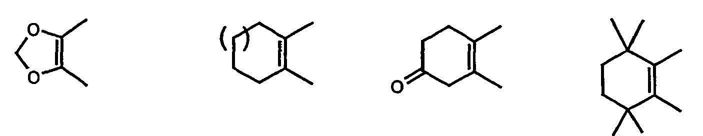

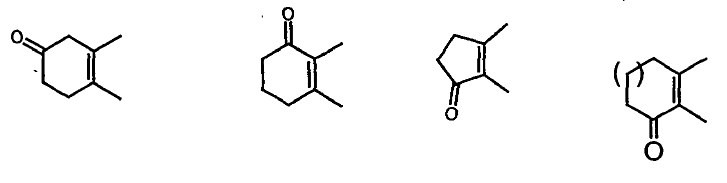

Vorzugsweise sind zwei benachbarte Kohlenstoffatome des Aromaten mit C₁₋₆-Alkylen zu einem 3 - 8gliedrigen, insbesondere C₃₋₄-Alkylen zu einem 5 - 6gliedrigen Ring verknüpft, der in beliebiger Position, insbesondere jedoch in 6-Stellung des Moleküls, substituiert ist.

Der Acylrest R⁴ leitet sich von geradkettigen oder verzweigten aliphatischen C₁₋₆-Carbonsäuren ab, wie beispielsweise Ameisensäure, Essigsäure, Propionsäure, Buttersäure, Trimethylessigsäure oder Capronsäure, oder von bekannten Benzolsulfonsäuren, die mit Halogen oder C₁₋₄-Alkyl substituiert sein können, sowie C₁₋₄-Alkansulfonsäuren, wie beispielsweise Methansulfonsäure, p-Toluolsulfonsäure.

Der Substituent n steht insbesondere für 1-6.

Weitere bevorzugte Ausführungsformen sind:
- R³, R⁴, R⁷ und R⁹: bedeuten jeweils Wasserstoff,
- r: bedeutet null,
- A: bedeutet C₁₋₆-Alkylen wie Methylen, Ethylen, Propylen, Butylen, Pentylen oder Hexylen,
- R¹: bedeutet -(CH R⁹)ₙ-NR⁷-B oder
- R¹ und R²: bilden gemeinsam einen 6gliedrigen Ring, der mit -(CH R⁹)ᵣ-NR⁷-B substituiert ist,
- B: bedeutet halogeniertes Alkyl, das mit -NHR⁷ oder mit C₆₋₁₀-Aryl substituiert sein kann, oder Cyclohexyl, das mit CF₃ substituiert ist.

Die Erfindung betrifft auch die Verwendung der erfindungsgemäßen Verbindungen zur Herstellung eines Arzneimittels zur Behandlung von Erkrankungen, die durch die Wirkung von Stickstoffmonoxid in pathologischen Konzentrationen hervorgerufen werden. Dazu zählen neurodegenerative Erkrankungen, inflammatorische Erkrankungen, Autoimmunerkrankungen, Herz-Kreislauf-Erkrankungen.

### Beispielsweise seien genannt:

Cerebrale Ischaemie, Hypoxie und andere neurodegenerative Erkrankungen, die mit Entzündungen in Verbindung gebracht werden wie Multiple Sklerose, Amyotrophe Lateralsklerose und vergleichbare sklerotische Erkrankungen, Morbus Parkinson, Huntington's Disease, Korksakoff's Disease, Epilepsie, Erbrechen, Schlafstörungen, Schizophrenie, Depression, Stress, Schmerz, Migräne, Hypoglykämie, Demenz wie z.B. Alzheimersche Krankheit, HIV-Demenz und Presenile Demenz.

Ferner eignen sie sich zur Behandlung von Krankheiten des Herz-Kreislauf-Systems und zur Behandlung autoimmuner und/oder inflammatorischer Erkrankungen wie Hypotension, ARDS (adult respiratory distress syndrome), Sepsis oder Septischer Schock, Rheumatoider Arthritis, Osteoarthritis, von insulinabhängiger Diabetes Mellitus (IDDM), entzündlicher Erkrankung des Beckens/Darms (bowel disease), von Meningitis, Glomerulonephritis, akute und chronische Lebererkrankungen, Erkrankungen durch Abstoßung (beispielsweise allogene Herz-, Nieren- oder Lebertransplantationen) oder entzündlichen Hautkrankheiten wie Psoriasis und andere.

Auf Grund ihres Wirkprofils eignen sich die erfindungsgemäßen Verbindungen sehr gut zur Inhibition der neuronalen NOS.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete Träger-, Hilfs- und/oder Zusatzstoffe enthält. Die Applikation kann oral oder sublingual als Feststoff in Form von Kapseln oder Tabletten oder als Flüssigkeit in Form von Lösungen, Suspensionen, Elixieren, Aerosolen oder Emulsionen oder rektal in Form von Suppositorien oder in Form von gegebenenfalls auch subcutan intramuskulär oder intravenös anwendbaren Injektionslösungen oder topisch oder intrathekal erfolgen. Als Hilfsstoffe für die gewünschte Arzneimittelformulierung sind die dem Fachmann bekannten inerten organischen und anorganischen Trägermaterialien geeignet wie z.B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 1 - 2000 mg, vorzugsweise 20 - 500 mg, wobei die Dosis als einmal zu verabreichende Einzeldosis oder unterteilt in zwei oder mehreren Tagesdosen gegeben werden kann.

Die NOS-inhibitorische Wirksamkeit der Verbindungen der Formel I und deren physiologisch verträglicher Salze kann nach den Methoden von Bredt und Snyder in Proc. Natl. Acad. Sci. USA 86, 9030 (1989)bestimmt werden.

Die Herstellung der erfindungsgemäßen Verbindungen erfolgt dadurch, daß man eine Verbindung der Formel II oder deren Salz 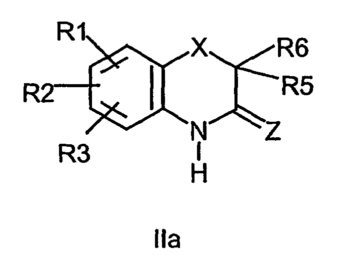 oder 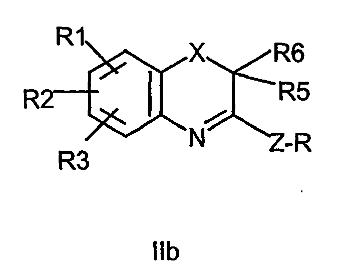 worin
R¹, R², R³, R⁵, R⁶ und X die obige Bedeutung haben, Z Sauerstoff oder Schwefel ist und R C₁₋₆-Alkyl bedeutet, mit Ammoniak oder primären Aminen umsetzt, wobei vorhandene Aminogruppen gegebenenfalls intermediär geschützt sind, und gewünschtenfalls anschließend acyliert, die Isomeren trennt oder die Salze bildet.

Die Umsetzung mit Ammoniak gelingt unter Druck in Autoklaven bei Ammoniaküberschuß bei tiefen Temperaturen (-78 °C) oder durch Rühren in mit Ammoniak gesättigten Methanol bei Raumtemperatur. Bevorzugt werden Thiolactame umgesetzt. Wird mit Aminen umgesetzt, so stellt man aus dem Lactam oder Thiolactam zunächst den Iminoether oder Iminothioether als Zwischenverbindung dar (z.B. mit Methyliodid oder Dimethylsulfat) und setzt diesen mit oder ohne Isolierung der Zwischenverbindung mit den entsprechenden Aminen oder deren Salzen um.

Als Aminoschutzgruppen sind beispielsweise Carbamate wie tert. Butyloxy-carbonyl, Benzyloxy-carbonyl oder Acetyl geeignet.

An den Vorstufen werden gewünschtenfalls Sulfide oxidiert, Ester verseift, Säuren verestert, Hydroxygruppen verethert oder acyliert, Amine acyliert, alkyliert, diazotiert, halogeniert, NO₂ eingeführt oder reduziert, mit Isocyanaten oder Isothiocyanaten umgesetzt, die Isomeren getrennt oder die Salze gebildet.

Die Verseifung einer Estergruppe kann basisch oder sauer erfolgen, indem man bei Raumtemperatur oder erhöhter Temperatur bis zur Siedetemperatur des Reaktionsgemisches in Gegenwart von Alkalihydroxiden in Ethanol oder anderen Alkoholen oder mittels Säuren wie z.B. Salzsäure hydrolysiert und ggf. Salze der Aminobenzoxazine oder -thiazine weiterverarbeitet.

Die Veresterung der Carbonsäure geschieht in an sich bekannter Weise mit Diazomethan oder dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Die Reduktion einer Estergruppe zum Alkohol erfolgt in an sich bekannter Weise mit DiBAH in geeignetem Lösungsmittel bei tiefen Temperaturen. Die reduktive Aminierung eines Ketons oder eines Benzaldehyds mit Amin unter Zugabe eines Borhydrides ergibt die entsprechenden Amine. Mit passend gewählten Diaminen erhält man nach Zugabe von gleichen oder verschiedenen Aldehyden bzw. Ketonen symmetrische oder unsymmetrische Aminoverbindungen.

Zusätzlich kann durch elektrophile aromatische Substitution eine Nitrogruppe oder Halogen, insbesondere Brom, eingeführt werden. Dabei entstehende Gemische können in üblicher Weise, auch mittels HPLC, getrennt werden. Wenn ein Nitril vorliegt, kann dieses nach bekannten Verfahren verseift werden oder in das entsprechende Amin, Tetrazol oder Amidoxim überführt werden oder es wird durch Angriff von substituierten Anilinen oder Aminen zu einem substituierten Amidin.

Die Friedel-Crafts Acylierung wird bei Lactamen vom Typ IIa erfolgreich angewandt, und anschließend kann selektiv das Lactam in das Thiolactam überführt oder das Acylierungsprodukt reduktiv aminiert werden.

Die Reduktion der Nitrogruppe oder ggf. der Cyanogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat oder Ameisensäure in bekannter Weise benutzt werden. Reduktionsmittel wie Zinn-II-chlorid können ebenso verwendet werden wie komplexe Metallhydride, eventuell in Gegenwart von Schwermetallsalzen. Es kann vorteilhaft sein, vor der Reduktion die Estergruppe wie in Formel V einzuführen. Für Nitrogruppen bewährt hat sich die Reduktion mit Zink oder Eisen in Essigsäure.

Wird eine einfache oder mehrfache Alkylierung einer Aminogruppe oder einer CHaciden Kohlenstoffposition gewünscht, so kann nach üblichen Methoden beispielsweise mit Alkylhalogeniden alkyliert werden.Gegebenenfalls ist Schutz der Lactamgruppe als Anion durch ein 2. Equivalent Base oder durch eine passende Schutzgruppe erforderlich.

Die Acylierung der Aminogruppe erfolgt in üblicher Weise beispielsweise mit einem Säurehalogenid oder Säureanhydrid gegebenenfalls in Gegenwart einer Base.

Die Einführung der Halogene Chlor, Brom oder Jod über die Aminogruppe kann beispielsweise auch nach Sandmeyer erfolgen, indem man die mit Nitriten intermediär gebildeten Diazoniumsalze mit Cu(l)chlorid oder Cu(l)bromid in Gegenwart der entsprechenden Säure wie Salzsäure oder Bromwasserstoffsäure umsetzt oder mit Kaliumjodid umsetzt.

Benzylalkohole lassen sich wie üblich mit Methansulfonylchlorid in die entsprechenden Mesylate bzw. Benzylhalogenide überführen.

Die Einführung einer NO₂-Gruppe gelingt durch eine Reihe von bekannten Nitrierungsmethoden. Beispielsweise kann mit Nitraten oder mit Nitroniumtetrafluoroborat in inerten Lösungsmitteln wie halogenierten Kohlenwasserstoffen oder in Sulfolan oder Eisessig nitriert werden. Möglich ist auch die Einführung z.B. durch Nitriersäure in Wasser oder konz. Schwefelsäure als Lösungsmittel bei Temperaturen zwischen -10 °C und 30 °C.

Die Isomerengemische können nach üblichen Methoden wie beispielsweise Kristallisation, Chromatographie oder Salzbildung in die Enantiomeren bzw. E/Z-Isomeren aufgetrennt werden. Die Enantiomere bzw. enantiomerenreinen Diastereomere können auch durch Chromatographie an chiralen Phasen sowie durch stereoselektive Synthese erhalten werden.

Die Herstellung der Salze erfolgt in üblicher Weise, indem man eine Lösung der Verbindung der Formel I - gegebenenfalls auch mit geschützten Aminogruppen - mit der äquivalenten Menge oder einem Überschuß einer Säure, die gegebenenfalls in Lösung ist, versetzt und den Niederschlag abtrennt oder in üblicher Weise die Lösung aufarbeitet.

Nucleophile Substitution von Benzylhalogeniden mit sekundären Aminen liefert die korrespondierenden Benzylamine.

Thiolactame der Formel IIa (Z = S) erhält man beispielsweise aus Lactamen mit Phosphorpentasulfid (P₄S₁₀) oder Lawesson-Reagenz (2,4-Bis(4-methoxphenyl)-1,3,2,4-dithiaphosphetan-2,4-disulfid) in geeigneten Lösungsmitteln und Verbindungen der Formell IIb können beispielsweise durch Umsetzung mit Meerwein-Reagenz (Trimethyloxoniumtetrafluoroborat) erhalten werden.

Die Erfindung betrifft auch die Zwischenverbindungen der Formel IIa und IIb und deren Salze 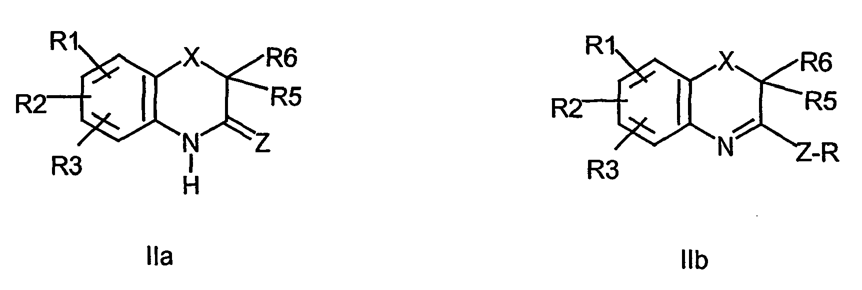 worin
R¹, R², R³, R⁵, R⁶ und X die obige Bedeutung haben, Z Sauerstoff oder Schwefel ist und R C₁₋₆-Alkyl bedeutet.

Die Herstellung pharmakologisch wirksamer Verbindungen aus den Zwischenprodukten erfolgt wie vorne beschrieben.

Die Herstellung der Verbindungen der Formel IIa kann beispielsweise dadurch erfolgen, daß man eine Verbindung der Formel III, 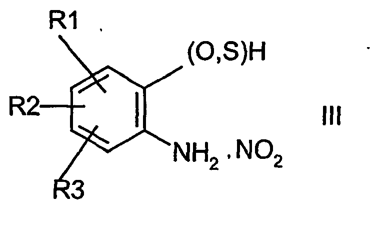 worin R¹ bis R³ die obige Bedeutung haben, mit einer Verbindung der Formel IV, 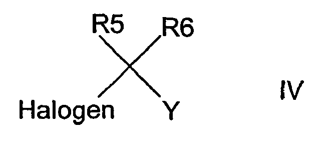 worin R⁵ und R⁶ die obige Bedeutung haben, und Y eine reaktive Carboxylgruppe ist wie Säurehalogenid, Nitril, Carbonsäureester umsetzt und gegebenenfalls reduktiv cyclisiert oder dadurch, daß man eine Verbindung der Formel V 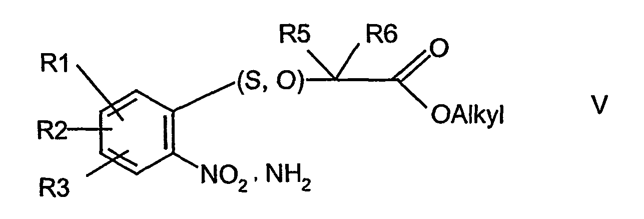 reduktiv cyclisiert.

Aromatische Thiole vom Typ III erhält man unter anderem wie in Chem. Pharm. Bull. 39, 2888 (1991) und der dort genannten Literatur beschrieben durch Umlagerung der entsprechenden Dimethylaminothiocarbamate.

Die Einführung der Substituenten R¹ bis R³ kann auf der Stufe der Verbindungen der Formel III oder 11 erfolgen.

Zur Herstellung von Verbindungen der Formel II kann der Aldehyd oder das Keton des entsprechenden 1,4-Benzoxazin-3-ons bzw. 1,4-Benzothiazin-3(4H)-ons reduktiv aminiert werden. Dies gelingt auch zweifach mit passend gewählten Diaminen. Diamine lassen sich auch umsetzen mit dem Aldehyd des 1,4-Benzoxazin-3-ons sowie gleichzeitig mit passend gewählten anderen Aldehyden. Wird die Einführung eines Heteroarylrestes Q gewünscht, so kann das entsprechende Halogenderivat nucleophil mit Amin substituiert werden. Ist eine primäre oder sekundäre Aminogruppe vorhanden, so kann es vorteilhaft sein, diese intermediär zu schützen, beispielsweise durch Einführung einer tert.-Butyloxycarbonylgruppe, die nach der Amidin-Bildung in üblicher Weise abgespalten wird.

Monoacylierte Diamine erhält man, wie in der Literatur (Synthesis 11; 917 (1988)) beschrieben, auch durch Umsetzung von Benzamiden mit Diamin unter Freisetzung von Ammoniak.

Soweit die Herstellung der Ausgangsverbindungen nicht beschrieben wird, sind diese bekannt und käuflich oder analog zu bekannten Verbindungen oder nach hier beschriebenen Verfahren herstellbar.

Neue Verbindungen wurden durch eine oder mehrere der folgenden Methoden charakterisiert: Schmelzpunkt, Massenspektroskopie, NMR. NMR Spektren wurden mit einem Bruker 300 MHz Gerät gemessen, die (deuterierten) Lösemittel sind wie folgt abgekürzt: CDCl₃ (Chloroform), DMSO (Dimethylsulfoxid). Verschiebungen sind in delta und ppm angegeben. Es bedeuten: m (Multiplett, mehrere Signale), s (Singulett), d (Dublett), dd (Doppeldublett .), t (Triplett), q (Quartett), H (Wasserstoffprotonen), J (Kopplungskonstante). Ferner bedeuten: THF (Tetrahydrofuran), DMF (N,N-Dimethylformamid), MeOH (Methanol), EE (Ethylacetat) ml (Milliliter), RT (Raumtemperatur). Alle Lösemittel sind p.A.Qualität, wenn nicht anders vermerkt. Alle Reaktionen werden unter Schutzgas ausgeführt, es sei denn, es handelt sich um wässrige Lösungen.

Nachfolgend wird die Darstellung einiger Vorstufen, Zwischenprodukte und Produkte exemplarisch beschrieben.

### Beispiel 1: (2R)-Methyl-3-amino-6-(2,2,3,3,3-pentafluorpropylamino-methyl)-2H-1,4-benzoxazin-dihydrochlorid

### a) (2R)-Methyl-3-oxo-6-(2,2,3,3,3-pentafluorpropylimino-methyl)-3,4-dihydro-2H-1,4-benzoxazin

1,15 g (5,98 mmol) (2R)-Methyl-3-oxo-6-aminomethyl-2H-1,4-benzoxazin, 885,7 mg (5,98 mmol) Pentafluorpropionaldehyd, 11,4 mg (0,06 mmol) para-Toluolsulfonsäure und 20 ml Benzol werden sieben Stunden am Wasserabscheider erhitzt. Nach dem Abkühlen wird bis zur Trockene einrotiert, und der Rückstand an Kieselgel (Laufmittel Essigsäureethylester/Hexan) chromatographiert. Es werden 712 mg (37%) der gewünschten Verbindung erhalten.

MS (Cl) m/e (relative Intensität) 340 (MNH₄⁺, 78), 193 (96), 176 (100)

### b) (2R)-Methyl-3-oxo-6-(2,2,3,3,3-pentafluorpropylamino-methyl)-3,4-dihydro-2H-1,4-benzoxazin

712 mg (2,21 mmol) (2R)-Methyl-3-oxo-6-(2,2,3,3,3-pentafluorpropylimino-methyl)-3,4-dihydro-2H-1,4-benzoxazin werden in 30 ml Methanol gelöst und mit 83,6 mg (2,21 mmol) Natriumborhydrid versetzt. Nach dem Rühren über Nacht bei Raumtemperatur wird der Ansatz auf Wasser gegeben und dreimal mit Essigsäureethylester geschüttelt. Die vereinigten organischen Phasen werden mit gesättigter Natriumchloridlösung gewaschen, getrocknet und einrotiert. Nach der Chromatographie an Kieselgel (Laufmittel Essigsäureethylester/Hexan) werden 396,7 mg (55%) der gewünschten Verbindung erhalten.

MS (CI) m/e (relative Intensität) 325 (M⁺, 77,5), 193 (86), 176 (100)

### c) (2R)-Methyl-3-thioxo-6-(2,2,3,3,3-pentafluorpropylamino-methyl)-3,4-dihydro-2H-1,4-benzoxazin

396 mg (1,22 mmol) (2R)-Methyl-3-oxo-6-(2,2,3,3,3-pentafluorpropylamino-methyl)-3,4-dihydro-2H-1,4-benzoxazin werden in 40 ml Dimethoxyethan mit 496 mg (1,22 mmol) Lawesson-Reagenz versetzt und über Nacht bei Raumtemperatur gerührt. Nach dem Abrotieren des Lösungsmittels wird der Rückstand chromatographiert (Laufmittel Essigsäureethylester/Hexan). Isoliert werden 234,5 mg (54%) des gewünschten Thiolactams.

MS (CI) m/e (relative Intensität) 341 (M⁺, 54,5), 309 (100), 209 (58,3), 192 (94)

### d) (2R)-Methyl-3-amino-6-(2,2,3,3,3-pentafluorpropylamino-methyl)-2H-1,4-benzoxazin-dihydrochlorid

234 mg (0,658 mmol) (2R)-Methyl-3-thioxo-6-(2,2,3,3,3-pentafluorpropylamino-methyl)-3,4-dihydro-2H-1,4-benzoxazin werden mit 10 ml einer 7M Lösung von Ammoniak in Methanol zwei Stunden bei Raumtemperatur gerührt. Anschließend wird der Ansatz bis zur Trockene eingeengt und der Rückstand in Dichlormethan gelöst. Nach Zugabe von 3 ml einer 4M Lösung von Chlorwasserstoff in Dioxan wird der Ansatz mit 20 ml Methyl-tert.-butylether versetzt und zwei Stunden bei Raumtemperatur gerührt. Die Menge des ausgefallenen Produkts beträgt 168,7 mg (65%).

MS (CI) m/e (relative Intensität) 324 (M⁺, 100), 192 (12,5), 172 (28,5)
In analoger Weise werden unter Verwendung der entsprechenden Ausgangsmaterialien synthetisiert:
(2R)-Methyl-3-amino-6-(2,2,2-trifluorethylamino-methyl)-2H-1,4-benzoxazin-dihydrochlorid,
(2R)-Methyl-3-amino-6-(2,2,3,3,3-pentafluorpropylamino-ethyl)-2H-1,4-benzoxazin-dihydrochlorid,
(2R)-Methyl-3-amino-6-(2,2,2-trifluorethylamino-ethyl)-2H-1,4-benzoxazin-dihydrochlorid,
(2R)-Methyl-3-amino-6-(2,2,3,3,4,4,4-heptafluorbutylamino-ethyl)-2H-1,4-benzoxazin-dihydrochlorid.

### Beispiel 2: (2R)-Methyl-3-amino-6-(4,4,4-trifluorbutylamino-methyl)-2H-1,4-benzoxazin-dihydrochlorid

### a) (2R)-Methyl-3-oxo-6-(4,4,4-trifluorbutylamino-methyl)-3,4-dihydro-2H-1,4-benzoxazin

780 mg (3,668 mmol) (2R)-Methyl-3-oxo-6-aminomethyl-2H-1,4-benzoxazin-hydrochlorid, 462,4 mg (3,668 mmol) 4,4,4-Trifluorbutyraldehyd und 371,2 mg (3,668 mmol) Triethylamin werden in einem Gemisch aus Methanol und Tetrahydrofuran (4:1) gelöst. Nach dreistündigem Rühren bei Raumtemperatur werden 75,2 mg Natriumborhydrid zugegeben. Nach dem Rühren über Nacht bei Raumtemperatur wird der Ansatz auf Wasser gegeben und dreimal mit Essigsäureethylester geschüttelt. Die vereinigten organischen Phasen werden mit gesättigter Natriumchloridlösung gewaschen, getrocknet und einrotiert. Nach der Chromatographie an Kieselgel (Laufmittel Dichlormethan/Isopropanol werden 608,8 mg (55%) der gewünschten Verbindung erhalten.

MS (CI) m/e (relative Intensität) 303 (M⁺, 100)

Nach der hier beschriebenen Weise werden aus den in WO99/12915 beschriebenen 2-Methyl-6-keto-6,7,8,9-tetrahydro-2H-naphth[2,3-b]-1,4-oxazin-3-on und 6-Formyl-2-methyl-2H-1,4-benzoxazin-3(4H)-on durch reduktive Aminierung mit den entsprechenden Aminen folgende Verbindungen hergestellt:
(2R)-Methyl-3-oxo-6-([4- amino-3,3,2,2-tetrafluoro-butyl-1- amino]-methyl)-2H-1,4-benzoxazin
(2R)-Methyl-3-oxo-6-([4- amino-3,3-difluoro-butyl-1- amino]-methyl)-2H-1,4-benzoxazin (6S)-(2R)-Methyl-6-(4,4,4-trifluorbutyl amino)-6,7,8,9-tetrahydro -2H-naphth[2,3-b]-1,4-oxazin-3-on
   und
(6R)-(2R)-Methyl-6-(4,4,4-trifluorbutyl-amino)-6,7,8,9-tetrahydro -2H-naphth[2,3-b]-1,4-oxazin-3-on und
(6S)-(2S)-Methyl-6-(4,4,4-trifluorbutyl-amino)-6,7,8,9-tetrahydro -2H-naphth[2,3-b]-1,4-oxazin-3-on
   und
(6R)-(2S)-Methyl-6-(4,4,4-trifluorbutyl-amino)-6,7,8,9-tetrahydro -2H-naphth[2,3-b]-1,4-oxazin-3-on

### b) (2R)-Methyl-3-oxo-6-(4,4,4-trifluorbutyl-tert.-butyloxycarbonyl-amino-methyl)-3,4-dihydro-2H-1,4-benzoxazin

608 mg (2,014 mmol) (2R)-Methyl-3-oxo-6-(4,4,4-trifluorbutylamino-methyl)-3,4-dihydro-2H-1,4-benzoxazin werden mit 439,9 mg (2,014 mmol) Di-tert.-Butyldicarbonat und 303 mg (2,995 mmol) Triethylamin in 15 ml Dichlormethan vier Stunden bei Raumtemperatur gerührt. Der Ansatz wird bis zur Trockene einrotiert und der Rückstand an Kieselgel (Laufmittel Essigsäureethylester/Hexan) gesäult. Isoliert werden 727,6 mg (99%) der gewünschten Verbindung.

MS (CI) m/e (relative Intensität) 420 (MNH₄⁺, 6,5), 403 (M⁺, 15), 364 (100), 303 (44), 176 (27)

In analoger Weise werden synthetisiert:
(2R)-Methyl-3-oxo-6-([4- tert.-butyloxycarbonyl-amino-3,3,2,2-tetrafluoro-butyl-1- tert.-butyloxycarbonyl-amino]-methyl)-2H-1,4-benzoxazin
(2R)-Methyl-3-oxo-6-([4- tert.-butyloxycarbonyl-amino-3,3-difluoro-butyl-1- tert.-butyloxycarbonyl-amino]-methyl)-2H-1,4-benzoxazin
(6S)-(2R)-Methyl-6-(4,4,4-trifluorbutyl tert.-butyloxycarbonyl-amino)-6,7,8,9-tetrahydro -2H-naphth[2,3-b]-1,4-oxazin-3-on
   und
(6R)-(2R)-Methyl-6-(4,4,4-trifluorbutyl-tert.-butyloxycarbonyl-amino)-6,7,8,9-tetrahydro -2H-naphth[2,3-b]-1,4-oxazin-3-on
   und
(6S)-(2S)-Methyl-6-(4,4,4-trifluorbutyl-tert: bufytoxycarbonyl-amino)-6,7,8,9-tetrahydro -2H-naphth[2,3-b]-1,4-oxazin-3-on
   und
(6R)-(2S)-Methyl-6-(4,4,4-trifluorbutyl-tert.-butyloxycarbonyl-amino)-6,7,8,9-tetrahydro -2H-naphth[2,3-b]-1,4-oxazin-3-on

### c) (2R)-Methyl-3-thioxo-6-(4,4,4-trifluorbutyl-tert.-butyloxycarbonyl-aminomethyl]93,4-dihydro-2H-1,4-benzoxazin

727 mg (1,986 mmol) (2R)-Methyl-3-oxo-6-(4,4,4-trifluorbutyl-tert.-butyloxycarbonylamino-methyl)3,4-dihydro-2H-1,4-benzoxazin werden in 7 ml Dimethoxyethan mit 802,7 mg Lawesson-Reagenz versetzt und über Nacht bei Raumtemperatur gerührt.

Nach dem Abrotieren des Lösungsmittels wird der Rückstand chromatographiert (Laufmittel Essigsäureethylester/Hexan). Isoliert werden 744 mg (98%) des gewünschten Thiolactams.

MS (FAB) m/e (relative Intensität) 419 (M⁺, 36,5), 217 (56), 109 (35), 91 (100)

In analoger Weise werden synthetisiert:
(2R)-Methyl-3-thioxo-6-([4- tert.-butyloxycarbonyl-amino-3,3,2,2-tetrafluoro-butyl-1- tert.-butyloxycarbonyl-amino]-methyl)-2H-1,4-benzoxazin
(2R)-Methyl-3-thioxo-6-([4- tert.-butyloxycarbonyl-amino-3,3-difluoro-butyl-1- tert.-butyloxycarbonyl-amino]-methyl)-2H-1,4-benzoxazin
(6S)-(2R)-Methyl-6-(4,4,4-trifluorbutyl tert.-butyloxycarbonyl-amino)-6,7,8,9-tetrahydro -2H-naphth[2,3-b]-1,4-oxazin-3-thion
   und
(6R)-(2R)-Methyl-6-(4,4,4-trifluorbutyl-tert.-butyloxycarbonyl-amino)-6,7,8,9-tetrahydro -2H-naphth[2,3-b]-1,4-oxazin-3-thion
   und
(6S)-(2S)-Methyl-6-(4,4,4-trifluorbutyl-tert.-butyloxycarbonyl-amino)-6,7,8,9-tetrahydro -2H-naphth[2,3-b]-1,4-oxazin-3-thion
   und
(6R)-(2S)-Methyl-6-(4,4,4-trifluorbutyl-tert.-butyloxycarbonyl-amino)-6,7,8,9-tetrahydro -2H-naphth[2,3-b]-1,4-oxazin-3-thion

### d) (2R)-Methyl-3-amino-6-(4,4,4-trifluorbutyl-tert.-butyloxycarbonyl-amino-methyl)-2H-1,4-benzoxazin

744 mg (1,946 mmol) (2R)-Methyl-3-thioxo-6-(4,4,4-trifluorbutyl-tert.-butyloxycarbonylamino-methyl)3,4-dihydro-2H-1,4-benzoxazin werden mit 10 ml einer 7M Lösung von Ammoniak in Methanol versetzt und über Nacht bei Raumtemperatur gerührt. Der Ansatz wird bis zur Trockene einrotiert und der Rückstand an Kieselgel chromatographiert (Laufmittel Dichlormethan/isopropanol). Isoliert werden 643,7 mg (85%) der gewünschten Verbindung.

MS (Cl) m/e (relative Intensität) 402 (M⁺, 100)

In analoger Weise werden synthetisiert:
(2R)-Methyl-3-amino-6-([4- tert.-butyloxycarbonyl-amino-3,3,2,2-tetrafluoro-butyl-1-tert.-butyloxycarbonyl-amino]-methyl)-2H-1,4-benzoxazin
(2R)-Methyl-3-amino-6-([4- tert.-butyloxycarbonyl-amino-3,3-difluoro-butyl-1- tert.-butyloxycarbonyl-amino]-methyl)-2H-1,4-benzoxazin
(6S)-(2R)-Methyl-6-(4,4,4-trifluorbutyl-tert.-butyloxycarbonyl-amino)-6,7,8,9-tetrahydro -2H-naphth[2,3-b]-1,4-oxazin-3-amin
   und
(6R)-(2R)-Methyl-6-(4,4,4-trifluorbutyl-tert.-butyloxycarbonyl-amino)-6,7,8,9-tetrahydro -2H-naphth[2,3-b]-1,4-oxazin-3-amin
   und
(6S)-(2S)-Methyl-6-(4,4,4-trifluorbutyl-tert.-butyloxycarbonyl-amino)-6,7,8,9-tetrahydro -2H-naphth[2,3-b]-1,4-oxazin-3-amin
   und
(6R)-(2S)-Methyl-6-(4,4,4-trifluorbutyl-tert.-butyloxycarbonyl-amino)-6,7,8,9-tetrahydro -2H-naphth[2,3-b]-1,4-oxazin-3-amin

### e) (2R)-Methyl-3-amino-6-(4,4,4-trifluorbutylamino-methyl)-2H-1,4-benzoxazindihydrochlorid

643 mg (1,485 mmol) (2R)-Methyl-3-amino-6-(4,4,4-trifluorbutyl-tert.-butyloxycarbonylamino-methyl)-2H-1,4-benzoxazin werden in 5 ml Dioxan gelöst und mit 5 ml einer 4M Lösung von Chlorwasserstoff in Dioxan versetzt. Nach dem Rühren über Nacht wird der Ansatz bis zur Trockene einrotiert, und der Rückstand mit einem Gemisch aus Dichlormethan/Methyl-tert.-butylether (1:1) ausgerührt.Nach dem Absaugen wird die erhaltene Verbindung an der Ölpumpe getrocknet.

MS (Cl) m/e (relative Intensität) 302 (M⁺, 100), 175 (19,5), 128 (20,3)

In analoger Weise wird unter Verwendung der entsprechenden Ausgangsmaterialien synthetisiert:
(2R)-Methyl-3-amino-6-(4,4,4-trifluorbutylamino-ethyl)-2H-1,4-benzoxazin-dihydrochlorid
(2R)-Methyl-3-amino-6-(2-fluorethylamino-methyl)-2H-1,4-benzoxazin-dihydrochlorid

In analoger Weise werden synthetisiert:
(2R)-Methyl-3-amino-6-([4-amino-3,3,2,2-tetrafluoro-butyl-1-amino]-methyl)-2H-1,4-benzoxazin-trihydrochlorid
(2R)-Methyl-3-amino-6-([4-amino-3,3-difluoro-butyl-1-amino]-methyl)-2H-1,4-benzoxazin-trihydrochlorid
(6S)-(2R)-Methyl-6-(4,4,4-trifluorbutylamino)-6,7,8,9-tetrahydro -2H-naphth[2,3-b]-1,4-oxazin-3-amin dihydrochlorid
   und
(6R)-(2R)-Methyl-6-(4,4,4-trifluorbutylamino)-6,7,8,9-tetrahydro -2H-naphth[2,3-b]-1,4-oxazin-3-amin dihydrochlorid
   und
(6S)-(2S)-Methyl-6-(4,4,4-trifluorbutylamino)-6,7,8,9-tetrahydro -2H-naphth[2,3-b]-1,4-oxazin-3-amin dihydrochlorid
   und
(6R)-(2S)-Methyl-6-(4,4,4-triffluorbutylamino)-6,7,8,9-tetrahydro -2H-naphth[2,3-b]-1,4-oxazin-3-amin dihydrochlorid

### Beispiel 3 : (2R)-Methyl-3-amino-6-[4,4,4-trifluorbut-(2RS)-yl-amino-methyl]-2H-1,4-benzoxazin-dihydrochlorid

### a) (2R)-Methyl-3-oxo-6-[4,4,4-trifluorbut-(2RS)-yl-amino-methyl]-3,4-dihydro-2H-1,4-benzoxazin

427,7 mg (2,615 mmol) racemisches 4,4,4-Trifluor-but-2-yl-amin-hydrochlorid, hergestellt nach K.-R. Gassen und W. Kirmse, Chem. Ber. 119, 2233 (1986), werden in einem Gemisch aus Methanol und Tetrahydrofuran (4:1) gelöst. Nach Zugabe von 264,6 mg (2,615 mmol) Triethylamin wird eine halbe Stunde bei Raumtemperatur gerührt. Anschließend werden 500 mg (2,615 mmol) (2R)-Methyl-3-oxo-6-formyl-2H-1,4-benzoxazin zugefügt und 15 Stunden bei Raumtemperatur gerührt. Nach Zugabe von 54,4 mg (1,438 mmol) Natriumborhydrid wird der Ansatz fünf Tage gerührt.Da auch nach dieser Zeit immer noch Ausgangsmaterial vorhanden ist, werden nochmals 54,4 mg (1,438 mmol) Natriumborhydrid zugegeben.Nach weiteren 18 Stunden bei Raumtemperatur wird der Ansatz auf Wasser/Sole gegeben und dreimal mit Essigsäureethylester extrahiert. Die vereinigten organischen Phasen werden mit gesättigter Natriumchloridlösung gewaschen, getrocknet und einrotiert. Nach der Chromatographie an Kieselgel (Laufmittel Dichlormethan/Ethanol/Ammoniakwasser) werden 581,6 mg (74%) der gewünschten Verbindung als Diastereomerengemisch erhalten.

MS (Cl) m/e (relative Intensität) 303 (M⁺, 100), 176 (55)

### b) (2R)-Methyl-3-oxo-6-[4,4,4-trifluorbut-(2RS)-yl-tert.-butyloxycarbonyl-aminomethyl]-3,4-dihydro-2H-1,4-benzoxazin

580 mg (1,918 mmol) (2R)-Methyl-3-oxo-6-[4,4,4-trifluorbut-(2RS)-yl-amino-methyl]-3,4-dihydro-2H-1,4-benzoxazin werden mit 502,3 mg (2,302 mmol) Di-tert.-Butyldicarbonat und 291,1 mg (2,877 mmol) Triethylamin in 11 ml Dichlormethan 20 Stunden bei Raumtemperatur gerührt. Der Ansatz wird mit 200 ml Dichlormethan verdünnt, mit gesättigter Natriumhydrogencarbonatlösung und mit Sole geschüttelt. Die organische Phase wird nach dem Trockenen abrotiert und der Rückstand an Kieselgel (Laufmittel Essigsäureethylester/Hexan) gesäult. Isoliert werden 751,8 mg (97%) der gewünschten Verbindung als Diasteremerengemisch.

MS (Cl) m/e (relative Intensität) 403 (M⁺, 9), 364 (100), 347 (75,8), 303 (30,5), 176 (69,5

### c) (2R)-Methyl-3-thioxo-6-[4,4,4-trifluorbut-(2RS)-yl-tert.-butyloxycarbonyl-amino-methyl]-3,4-dihydro-2H-1,4-benzoxazin

750 mg (1,864 mmol) (2R)-Methyl-3-oxo-6-[4,4,4-trifluorbut-(2RS)-yl-tert.-butyloxycarbonyl-amino-methyl]-3,4-dihydro-2H-1,4-benzoxazin werden in 29 ml Dimethoxyethan mit 829,3 mg (2,05 mmol) Lawesson-Reagenz versetzt und über Nacht bei Raumtemperatur gerührt. Der Ansatz wird filtriert und der Filterrückstand mit Dichlormethan gewaschen Nach dem Abrotieren des Lösungsmittels wird der Rückstand chromatographiert (Laufmittel Essigsäureethylester/Hexan). Isoliert werden 754,2 mg (97%) des gewünschten Thiolactams als Diastereomerengemisch.

MS (CI) m/e (relative Intensität) 419 (M⁺, 21), 363 (100)

### d) (2R)-Methyl-3-amino-6-[4,4,4-trifluorbut-(2RS)-yl-tert.-butyloxycarbonyl-aminomethyl]-2H-1,4-benzoxazin

754 mg (1,802 mmol) (2R)-Methyl-3-thioxo-6-[4,4,4-trifluorbut-(2RS)-yl-tert.-butyloxycarbonyl-amino-methyl]-3,4-dihydro-2H-1,4-benzoxazin werden mit 29 ml einer 7M Lösung von Ammoniak in Methanol versetzt und zwei Stunden bei Raumtemperatur gerührt. Der Ansatz wird mit 20 ml Toluol versetzt und bis zur Trockene einrotiert. Der Rückstand wird an Kieselgel chromatographiert (Laufmittel Dichlormethan/Isopropanol/Ammoniakwasser). Isoliert werden 553,5 mg (77%) der gewünschten Verbindung als Diastereomerengemisch.

MS (Cl) m/e (relative Intensität) 402 (M⁺, 100), 345 (29,8)

### e) (2R)-Methyl-3-amino-6-[4,4,4-trifluorbut-(2RS)-yl-amino-methyl)-2H-1,4-benzoxazin-dihydrochlorid

550 mg (1,37 mmol) (2R)-Methyl-3-amino-6-[4,4,4-trifluorbut-(2RS)-yl-tert.-butyloxycarbonyl-amino-methyl]-2H-1,4-benzoxazin werden in 3 ml Dioxan gelöst und mit 4 ml einer 4M Lösung von Chlorwasserstoff in Dioxan versetzt. Nach anderthalb Stunden Rühren bei Raumtemperatur beginnt sich ein halbkristalliner Niederschlag abzuscheiden. Nach drei Stunden wird das überstehende Lösungsmittel dekantiert, und es werden 20 ml Toluol zugegeben. Nach dem Einrotieren des Toluols wird der Rückstand an der Ölpumpe getrocknet. Isoliert werden 379 mg (74%) der gewünschten Verbindung als Diastereomerengemisch.

MS (Cl) m/e (relative Intensität) 302 (M⁺, 100), 175 (89,5)

### Beispiel 4: (2R)-Methyl-3-amino-6-[(3R)-Fluor-3-phenyl-prop-(2R)-yl-aminomethyl]-2H-1,4-benzoxazin-dihydrochlorid

### a) [(1S)-Phenyl-(2R)-(2,5-dimethylpyrrol-1-yl)]-propan-1-ol

3,97 g (26,257mmol) (1S, 2R)-(+)-Norephedrin werden mit 3,02 g (26,257 mmol) Hexan-2,5-dion in 15 ml Methanol vier Stunden am Rückfluß gekocht. Nach dem Verdünnen mit 150 ml Essigsäureethylester, wird die organische Phase zweimal mit gesättigter Natriumhydrogencarbonat- und einmal mit gesättigter Kochsalzlösung gewaschen. Nach dem Trocknen und Einrotieren wird der Rückstand an Kieselgel (Laufmittel Essigsäureethylester/Hexan) chromatographiert. Isoliert werden 5,64 g (94%) der gewünschten Verbindung.

MS (Cl) m/e (relative Intensität) 230 (M⁺, 100), 122 (10)

### b) 1-[(1 R)-Methyl-(2R)-fluoro-2-phenyl-eth-1-yl]-2,5-dimethyl-pyrrol

4,64 g (20,235 mmol) [(1S)-Phenyl-(2R)-(2,5-dimethylpyrrol-1-yl)]-propan-1-ol und 8,96 ml (60,705 mmol) DBU werden in 400 ml Toluol gelöst. Nach der Zugabe von von 9,19 g (30,392 mmol) Perfluorbutansulfonsäurefluorid (leichte Erwärmung auf 30°C) wird der Ansatz vier Stunden bei Raumtemperatur gerührt. Das DBU wird im Scheidetrichter abgetrennt und die organische Phase bis zur Trockene einrotiert.Nach der Chromatographie an Kieselgel (Laufmittel Essigsäureethylester/Hexan) werden 2,96 g (63%) der gewünschten Verbindung erhalten.

MS (Cl) m/e (relative Intensität) 232 (M⁺, 100), 212 (70,5), 122 (5,5)

### c) (3R)-Fluor-3-phenyl-prop-(2R)-yl-amin

2,96 g (12,797 mmol) 1-[(1 R)-Methyl-(2R)-fluor-2-phenyl-eth-1-yl]-2,5-dimethyl-pyrrol, 8,92 g (127,97 mmol) Hydroxylaminhydrochlorid, 4,45 g (79,37 mmol) Kaliumhydroxyd, 64 ml Ethanol und 25 ml Wasser werden über Nacht am Rückfluß gekocht. Das Ethanol wird abrotiert und der Rückstand mit 2M Salzsäure auf pH 2 gebracht. Nach viermaligem Extrahieren mit Diethylether wird die wässrige Phase mit Kaliumhydroxid auf pH 9 gebracht und dreimal mit Essigsäureethylester extrahiert. Die vereinigten Essigsäureethylesterextrakte werden getrocknet und das Lösungsmittel abrotiert. Zurück bleiben 772,7 mg (39%) der gewünschten Verbindung.

MS (Cl) m/e (relative Intensität) 154 (M⁺, 25,5), 145 (100)

### d) (2R)-Methyl-3-oxo-6-[(3R)-fluor-3-phenyl-prop-(2R)-yl-amino-methyl]-3,4-dihydro-2H-1,4-benzoxazin

1,02 g (6,658 mmol) (3R)-Fluor-3-phenyl-prop-(2R)-yl-amin und 1,27 g (6,658 mmol) (2R)-Methyl-3-oxo-6-formyl-3,4-dihydro-2H-1,4-benzoxazin in 25 ml eines Gemisches aus Methanol und Tetrahydrofuran (4:1) gelöst. Nach dem Rühren über Nacht werden 136, mg Natriumborhydrid zugegeben und weitere zwei Stunden gerührt Der Ansatz wird auf auf Wasser gegeben und viermal mit Essigsäureethylester extrahiert. Die vereinigten organischen Phasen werden mit gesättigter Natriumchloridlösung gewaschen, getrocknet und einrotiert. Nach der Chromatographie an Kieselgel (Laufmittel Essigsäureethylester/Hexan) werden 1,92 g (88%) der gewünschten Verbindung erhalten.

MS (El) m/e (relative Intensität) 219 (42,8), 176 (100)

### e) (2R)-Methyl-3-oxo-6-[(3R)-fluor-3-phenyl prop-(2R)-yl-tert.-butyloxycarbonyl-amino-methyl]-3,4-dihydro-2H-1,4-benzoxazin

1,92 g (5,847 mmol) 2R)-Methyl-3-oxo-6-[(3R)-fluor-3-phenyl prop-(2R)-yl-aminomethyl]-3,4-dihydro-2H-1,4-benzoxazin werden mit 1,279 g (5,847 mmol) Di-tert.-Butyldicarbonat und 1,21 ml (8,705 mmol) Triethylamin in 60 ml Dichlormethan über Nacht bei Raumtemperatur gerührt. Der Ansatz wird zur Trockene einrotiert und der Rückstand an Kieselgel (Laufmittel Essigsäureethylester/Hexan) chromatographiert. Isoliert werden 2,35 g (94%) der gewünschten Verbindung.

MS (C1) m/e (relative Intensität) 429 (M⁺, 15), 353 (40,5), 329 (52), 219 (46), 176 (100)

### f) (2R)-Methyl-3-thioxo-6-[(3R)-fluor-3-phenyl prop-(2R)-yl-tert.-butyloxycarbonyl-amino-methyl]-3,4-dihydro-2H-1,4-benzoxazin

2,35 g (5,485 mmol) (2R)-Methyl-3-oxo-6-[(3R)-fluor-3-phenyl prop-(2R)-yl-tert.-butyloxycarbonyl-amino-methyl]-3,4-dihydro-2H-1,4-benzoxazin werden in 230 ml Dimethoxyethan mit 2,22 g Lawesson-Reagenz versetzt und 65 Stunden bei Raumtemperatur gerührt. Das Lösungsmittel wird abrotiert, und der Rückstand an Kieselgel (Laufmittel Essigsäureethylester/Hexan) chromatographiert. Es werden 2,06 g (84%) der gewünschten Verbindung erhalten.

MS (Cl) m/e (relative Intensität) 445 (M⁺, 25), 389 (100), 192 (44,5)

### g) (2R)-Methyl-3-amino-6-[(3R)-fluor-3-pheriyl prop-(2R)-yl-tert.-butyloxycarbonyl-amino-methyl]-2H-1,4-benzoxazin

2,06 g (4,634 mmol) (2R)-Methyl-3-thioxo-6-[(3R)-fluor-3-phenyl prop-(2R)-yl-tert.-butyloxycarbonyl-amino-methyl]-3,4-dihydro-2H-1,4-benzoxazin werden mit 40 ml einer 7 M Lösung von Ammoniak in Methanol versetzt und über Nacht bei Raumtemperatur gerührt. Nach dem Versetzen des Ansatzes mit Toluol, wird das Lösungsmittel abrotiert und der Rückstand an Kieselgel (Laufmittel Dichlormethan/lsopropanol) chromatographiert. Isoliert werden 1,43 g (72%) der gewünschten Verbindung.

MS (Cl) m/e (relative Intensität) 428 (M⁺, 100),

### h) (2R)-Methyl-3-amino-6-[(3R)-fluor-3-phenyl prop-(2R)-yl]-amino-methyl]-2H-1,4-benzoxazin-dihydrochlorid

1,43 g (3,345 mmol) (2R)-Methyl-3-amino-6-[(3R)-fluor-3-phenyl prop-(2R)-yl-tert.-butyloxycarbonyl-amino-methyl]-2H-1,4-benzoxazin werden in 10 ml Dioxan gelöst und mit 10 ml einer 4M Lösung von Chlorwasserstoff in Dioxan versetzt. Nach dem Rühren über Nacht wird der Ansatz bis zur Trockenen einrotiert, und der Rückstand mit einer Mischung aus Dichlormethan/Methyl-tert.-butylether ausgerührt. Das kristalline Produkt wird abgesaugt: 1,12 g (84%).

MS (Cl) m/e (relative Intensität) 328 (M⁺, 100), 308 (24,5), 218 (22), 175 (69)

In analoger Weise wird unter Verwendung der entsprechenden Ausgangsmaterialien synthetisiert:
(2R)-Methyl-3-amino-6-[(3S)-fluor-3-phenyl prop-(2S)-yl-amino-methyl]-2H-1,4-benzoxazin-dihydrochlorid

### Beispiel 5: (2R)-Methyl-3-amino-6-{[(1SR, 3RS)-3-(trifluormethyl)-cyclohexyl]-amino-methyl]}-2H-1,4-benzoxazin-dihydrochlorid

### a) 3-(Trifluormethyl)-cyclohexanon-oxim

5 g (30,095 mmol) 3-(Trifluormethyl)-cyclohexanon werden in 30 ml Ethanol mit 2,51 g (36,08 mmol) Hydroxylamin-hydrochlorid, 3,95 g (48,081 mmol) Natriumacetat und 15,7 ml Wasser sechs Stunden bei 40°C gerührt. Die Mischung wird mit Wasser verdünnt und dreimal mit Essigsäureethylester geschüttelt. Die vereinigten organischen Extrakte werden neutralgewaschen und das Lösungsmittel nach dem Trocknen abrotiert. Der Rückstand wird an Kieselgel (Laufmittel Essigsäureethylester/Hexan) chromatographiert. Es werden 5,78 g (>100%) der gewünschten, noch leicht verunreinigten Verbindung isoliert.

MS (CI) m/e (relative Intensität) 182 (M⁺, 100)

### b) (1RS, 3RS) und (1SR, 3RS)-3-Trifluormethyl-cyclohexylamin

5,28 g (29,142 mmol) 3-(Trifluormethyl)-cyclohexanon-oxim werden in 200 ml Methanol mit 7,71 ml konzentrierter Salzsäure und 476,9 mg Pd/C (10%ig) versetzt und über Nacht hydriert. Der Katalysator wird über einen Glasfaserfilter abgesaugt und das Filtrat bis zur Trockene einrotiert. Der Rückstand wird mit 0,5 N Salzsäure aufgenommen und zweimal mit Methyl-tert.-butylether extrahiert. Die wässrige Phase wird mit Kaliumhydroxid auf pH 9 gebracht, auf Extrelut gegeben und mit Dichlormethan eluiert. Das Eluat wird nach dem Einrotieren an Kieselgel (Laufmittel Essigsäureethylester/Hexan) chromatographiert. Es werden die beiden diastereomeren Amine jeweils als Racemat erhalten.

MS (CI) m/e (relative Intensität) 224 (100), 208 (78), 182 (81), 168 (M⁺, 96,5)

### c) (2R)-Methyl-3-oxo-6-{(E/Z)-[(1SR, 3RS)-3-(trifluormethyl)-cyclohexyl]-imino-methyl]}-3,4-dihydro-2H-1,4-benzoxazin

385,4 mg (2,016 mmol) (1SR, 3RS)-3-Trifluormethyl-cyclohexylamin, 337 mg (2,016 mmol) (2R)-Methyl-3-oxo-6-formyl-2H-3,4-dihydro-1,4-benzoxazin und 38 mg (0,2 mmol) para-Toluolsulfonsäure werden in 15 ml Benzol über Nacht am Wasserabscheider gekocht. Der nach dem Abkühlen ausgefallene Feststoff wird abgesaugt und mit dem nach Chromatographie des Rückstandes (Kieselgel, Essigsäureethylester/Hexan) erhaltenen Produkt vereinigt. Die Ausbeute beträgt 584,7 mg (85%).

MS (CI) m/e (relative Intensität) 341 (M⁺, 100)

### d) (2R)-Methyl-3-oxo-6-{[(1 SR, 3RS)-3-(trifluormethyl)-cyclohexyl]-amino-methyl]}-3,4-dihydro-2H-1,4-benzoxazin

360,6 mg (1,06 mmol) (2R)-Methyl-3-oxo-6-{(E/Z)-[(1SR, 3RS)-3-(trifluormethyl)-cyclohexyl]-imino-methyl]}-3,4-dihydro-2H-1,4-benzoxazin werden in 11 ml Methanol gegeben und unter Zugabe von 0,29 ml konzentrierter Salzsäure und 17,3 mg Pd/C (10%ig) über Nacht hydriert.
Die Aufarbeitung erfolgt wie in b) beschrieben. Isoliert werden 165,9 mg (46%) der gewünschten Verbindung.

MS (Cl) m/e (relative Intensität) 343 (M⁺, 92), 176 (100)

### e) (2R)-Methyl-3-oxo-6-{[(1SR, 3RS)-3-(trifluormethyl)-cyclohexyl]-tert.-butyloxycarbonyl-amino-methyl]}-3,4-dihydro-2H-1,4-benzoxazin

166 mg (0,485 mmol) (2R)-Methyl-3-oxo-6-{[(1SR, 3RS)-3-(trifluormethyl)-cyclohexyl]-amino-methyl]}-3,4-dihydro-2H-1,4-benzoxazin werden in zehn ml Dichlormethan gelöst, mit 0,1 ml (0,722 mmol) Triethylamin und 105,7 mg (0,485 mmol) Di-tert.-Butyldicarbonat versetzt und über Nacht bei Raumtemperatur gerührt. Nach dem Einrotieren bis zur Trockene wird der Rückstand wie üblich an Kieselgel (Laufmittel Essigsäureethylester/Hexan) chromatographiert, Zurück bleiben 212,2 mg (99%) des gewünschten Produkts.

MS (CI) m/e (relative Intensität) 443 (M⁺, 30,5), 386 (70,5), 343 (66), 176 (100)

### f) (2R)-Methyl-3-thioxo-6-{[(1SR, 3RS)-3-(trifluormethyl)-cyclohexyl]-tert.-butyloxycarbonyl-amino-methyl]}-3,4-dihydro-2H-1,4-benzoxazin

212 mg (0,480 mmol) (2R)-Methyl-3-oxo-6-{[(1SR, 3RS)-3-(trifluormethyl)-cyclohexyl]-tert.-butyloxycarbonyl-amino-methyl]}-3,4-dihydro-2H-1,4-benzoxazin werden in 15 ml Dimethoxyethan mit 194,8 mg Lawesson-Reagenz versetzt und über Nacht bei Raumtemperatur gerührt. Nach dem Abrotieren des Lösungsmittels wird der Rückstand an Kieselgel (Laufmittel Essigsäureethyester/Hexan) chromatographiert. Die gewünschte Verbindung wird in 96% Ausbeute (210,9 mg) erhalten.

MS (CI) m/e (relative Intensität) 459 (M⁺, 10), 403 (82), 359 (100), 192 (81)

### g) (2R)-Methyl-3-amino-6-{[(1SR, 3RS)-3-(trifluormethyl)-cyclohexyl]-tert.-butyloxycarbonyl-amino-methyl]}-2H-1,4-benzoxazin

210,9 mg (2R)-Methyl-3-thioxo-6-{[(1SR, 3RS)-3-(trifluormethyl)-cyclohexyl]-tert.-butyloxycarbonyl-amino-methyl]}-3,4-dihydro-2H-1,4-benzoxazin werden mit 10 ml einer 7M Lösung von Ammoniak in Methanol versetzt und über Nacht bei Raumtemperatur gerührt. Nach dem Versetzen des Ansatzes mit Toluol wird bis zur Trockene einrotiert und der Rückstand an Kieselgel (Laufmittel Essigsäureethylester/Hexan) chromatographiert. Es werden 156,3 mg (77%) der gewünschten Verbindung erhalten.

MS (Cl) m/e (relative Intensität) 442 (M⁺, 100), 385 (40), 175 (22,5)

### h) (2R)-Methyl-3-amino-6-{[(1 SR, 3RS)-3-(trifluormethyl)-cyclohexyl]-aminomethyl]}-2H-1,4-benzoxazin-dihydrochlorid

156,3 mg (0,354 mmol) (2R)-Methyl-3-amino-6-{[(1SR, 3RS)-3-(trifluormethyl)-cyclohexyl]-tert.-butyloxycarbonyl-amino-methyl]}-2H-1,4-benzoxazin werden in einem ml Dioxan gelöst und mit 2 ml einer 4M Lösung von Chlorwasserstoff in Dioxan versetzt. Nach dem Rühren über Nacht wird bis zur Trockene eingeengt und der Rückstand mit 10 ml eines Gemisches aus Dichlormethan und Methyl-tert.-butyl-ether (1:1) ausgerührt. Das ausgefallene Produkt wird abgesaugt und an der Ölpumpe getrocknet. Die Ausbeute beträgt 101,2 mg (69%).

MS (Cl) m/e (relative Intensität) 342 (M⁺, 78), 175 (100)

Analog werden synthetisiert:
(2R)-Methyl-3-amino-6-{[(1RS, 3RS)-3-(trifluormethyl)-cyclohexyl]-amino-methyl]}-2H-1,4-benzoxazin-dihydrochlorid
(2R)-Methyl-3-amino-6-{[(1SR, 2RS)-2-(trifluormethyl)-cyclohexyl]-amino-methyl]}-2H-1,4-benzoxazin-dihydrochlorid
(2R)-Methyl-3-amino-6-{[(1RS, 2RS)-2-(trifluormethyl)-cyclohexyl]-amino-methyl]}-2H-1,4-benzoxazin-dihydrochlorid

## Patentansprüche

1. Verbindungen der Formel I, deren Tautomere und E- und Z-Isomere, S- und R-Enantiomere, Diastereomere, Racemate und Gemische derselben, und Salze 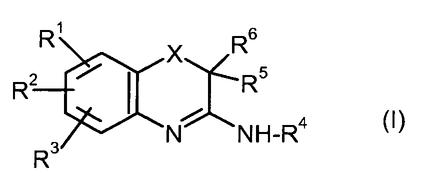 worin
X O oder S,
R¹ -(CHR⁹)ₙ-NR⁷-B,
R² Wasserstoff oder
R¹ und R² gemeinsam mit zwei benachbarten Kohlenstoffatomen einen 5-, 6-, 7- oder 8-gliedrigen Ring bilden, der monocyclisch oder bicyclisch, gesättigt oder ungesättigt ist und bei dem 1 oder 2 CH₂-Gruppen durch Sauerstoff oder Carbonyl ersetzt sein können und der mit -(CHR⁹)ᵣ-NR⁷-B substituiert ist und mit C₁₋₄-Alkyl substituiert sein kann,
R³ Wasserstoff, Halogen, NO₂, Cyano, CF₃, -OCF₃, -S-R⁹, -O-R⁹, C₃₋₇-Cycloalkyl, -NR⁹-C(=NR¹⁰)-R¹¹, -NH-CS-NR¹²R¹³, NH-CO-NR¹²R¹³, -SO₂NR¹²R¹³, -CO-NR¹²R¹³, -CO-R¹⁴, NR¹⁵R¹⁶, C₆₋₁₀Aryl, das gegebenenfalls mit Halogen, Cyano, C₁₋₄-Alkyl, -S-R⁹ oder -O-R⁹ substituiert ist,
5- oder 6 gliedriges Heteroaryl mit 1 - 4 Sauerstoff-, Schwefel- oder Stickstoffatomen
C₁₋₆-Alkyl, das gegebenenfalls mit Halogen, OR⁹, SR⁹, NR¹²R¹³, =NR¹², =NOC₁₋₆-Alkyl, =N-NHAryl, Phenyl, C₃₋₇-Cycloalkyl oder 5- oder 6 gliedrigem Heteroaryl substituiert ist,
C₂₋₆-Alkenyl, das gegebenenfalls mit Halogen, CONH₂, C≡N oder Phenyl substituiert ist,
C₂₋₆-Alkinyl, das gegebenenfalls mit Halogen, CONH₂, C≡N oder Phenyl substituiert ist,
R⁴ Wasserstoff oder Acyl,
R⁵ und R⁶ unabhängig voneinander Wasserstoff, C₃₋₇-Cycloalkyl, Phenyl, C₁₋₆-Alkyl, C₂₋₆-Alkenyl- oder C₂₋₆-Alkynylreste, die jeweils substituiert sein können mit Halogen, OH, O-C₁₋₆-Alkyl, SH, S-C₁₋₆-Alkyl, NR¹⁵R¹⁶, 5- oder 6-gliedriges Hereroaryl mit 1-3 N-, O- oder S-Atomen, Phenyl oder C₃₋₇-Cycloalkyl,
R⁷ Wasserstoff, C₁₋₆-Alkyl, das mit Phenyl substituiert sein kann, COOC₁₋₆-Alkyl oder COC₁₋₆-Alkyl,
R⁸ Wasserstoff,
A geradkettiges oder verzweigtes -C₁₋₆-Alkylen, geradkettiges oder verzweigtes C₂₋₆- Alkenylen,
B halogeniertes C₁₋₈-Alkyl, das mit C₆₋₁₀-Aryl substituiert sein kann, wobei der Arylrest mit Halogen, Cyano, OH, C₁₋₄-Alkoxy, C₁₋₄-Alkyl, CF₃ und -OCF₃ substituiert sein kann, C₃₋₈Alkinyl, das mit C₆₋₁₀-Aryl substituiert sein kann, -(CH₂)_{P}-C₆₋₁₀-Aryl, das mit -OCF₃ substituiert ist, oder C₃₋₇-Cycloalkyl, das mit CF₃ substituiert ist,
n und r 0, 1 bis 6,
p 0 bis 6 ,
R⁹ und R¹⁰ Wasserstoff oder C₁₋₆-Alkyl,
R¹¹ C₁₋₆-Alkyl, -NH₂, -NH-CH₃, -NH-CN, gegebenenfalls mit Halogen, C₁₋₄-Alkyl oder CF₃ substituiertes C₆₋₁₀-Aryl oder gegebenenfalls mit Halogen, C₁₋₄-Alkyl oder CF₃ substituiertes 5- oder 6 gliedriges Heteroaryl mit 1 bis 4 Stickstoff-, Schwefel- oder Sauerstoffatomen,
R¹² und R¹³ Wasserstoff, C₁₋₆-Alkyl, gegebenenfalls mit Halogen oder C₁₋₄-Alkyl substituiertes Phenyl, gegebenenfalls mit Halogen oder C₁₋₄-Alkyl substituiertes Benzyl oder C₃₋₇-Cycloalkyl,
R¹⁴ Wasserstoff, Hydroxy, C₁₋₆-Alkoxy, Phenyl, gegebenenfalls mit CO₂H, CO₂C₁₋₆-Alkyl, Hydroxy, C₁₋₄-Alkoxy, Halogen, NR¹⁵R¹⁶, CONR¹²R¹³ oder Phenyl substituiertes C₁₋₆-Alkyl oder gegebenenfalls mit Phenyl, Cyano, CONR¹²R¹³ oder CO₂C₁₋₄-Alkyl substituiertes C₂₋₆-Alkenyl,
R¹⁵ und R¹⁶ Wasserstoff, C₁₋₆-Alkyl, gegebenenfalls mit Halogen oder C₁₋₄-Alkyl substituiertes Phenyl oder gegebenenfalls mit Halogen oder C₁₋₄-Alkyl substituiertes Benzyl oder
R¹⁵, R¹⁶ gemeinsam mit dem Stickstoffatom einen gesättigten 5-, 6- oder 7gliedrigen Ring bilden, der ein weiteres Stickstoff-, Sauerstoff oder Schwefelatom enthalten und substituiert sein kann mit C₁₋₄-Alkyl- oder einem gegebenenfalls mit Halogen substituierten Phenyl-, Benzyl- oder Benzoylrest
bedeuten.

2. Verbindungen gemäß Anspruch 1, worin R⁶ Wasserstoff ist.

3. Verbindungen nach Anspruch 1 bis 2, worin R⁵ C₁₋₆-Alkyl ist.

4. Verbindungen nach Anspruch 1 bis 3, worin R⁴ Wasserstoff ist.

5. Verbindungen nach Anspruch 1 bis 4, worin R³ Wasserstoff ist.

6. Verbindungen nach Anspruch 1, worin A geradkettiges oder verzweigtes C₁₋₆-Alkylen ist.

7. Verbindungen gemäß einem der Ansprüche 1-6, nämlich
(2R)-Methyl-3-amino-6-(2,2,3,3,3-pentafluorpropylamino-methyl)-2H-1,4-benzoxazin-dihydrochlorid
(2R)-Methyl-3-amino-6-(4,4,4-trifluorbutylamino-methyl)-2H-1,4-benzoxazin-dihydrochlorid
(2R)-Methyl-3-amino-6-[4,4,4-trifluorbut-(2RS)-yl-amino-methyl]-2H-1,4-benzoxazin-dihydrochlorid
(2R)-Methyl-3-amino-6-[(3R)-Fluor-3-phenyl-prop-(2R)-yl-amino-methyl]-2H-1,4-benzoxazin-dihydrochlorid

8. Arzneimittel enthaltend eine Verbindung gemäß einem der Ansprüche 1 bis 7 und einen oder mehrere pharmazeutisch übliche Träger- oder Hilfsstoffe.

9. Verwendung einer Verbindung gemäß einem der Ansprüche 1 bis 7 zur Herstellung eines Arzneimittels zur Behandlung einer Erkrankung, die ausgewählt ist aus der Gruppe, bestehend aus neurodegenerativen Erkrankungen, Autoimmunerkrankungen, inflammatorischen Erkrankungen, und Herz-Kreislauf-Erkrankungen.

10. Verwendung gemäß Anspruch 9 zur Behandlung neurodegenerativer Erkrankungen.

11. Verwendung gemäß Anspruch 9, wobei die Erkrankung ausgewählt ist aus der Gruppe, bestehend aus Cerebraler Ischämie, Hypoxie, Multipler Sklerose, Amyotropher Lateralsklerose und vergleichbarer sklerotischer Erkrankungen, Morbus Parkinson, Huntington's Disease, Korsakoff's Disease, Epilepsie, Erbrechen, Schlafstörungen, Schizophrenie, Depression, Stress, Schmerz, Migräne, Hypoglykämie, Demenz, Hypotension, ARDS (adult respiratory distress syndrome), Sepsis, Septischer Schock, Rheumatoider Arthritis, Osteoarthritis, insulinabhängigem Diabetes mellitus (IDDM), entzündlicher Erkrankung des Beckens/Darms (bowel disease), Meningitis, Glomerulonephritis, akuter und chronischer Lebererkrankungen, Erkrankungen durch Abstoßung (beispielsweise allogene Herz-, Leber- und Nierentransplantationen), entzündlicher Hauterkrankungen, und Psoriasis.

12. Verwendung gemäß Anspruch 11, wobei die Demenz ausgewählt ist aus der Gruppe, bestehend aus Alzheimerscher Krankheit, HIV-Demenz, und Preseniler Demenz.

13. Verbindungen gemäß einem der Ansprüche 1 bis 7 zur therapeutischen Verwendung.

14. Verfahren zur Herstellung einer Verbindung der Formel I nach Anspruch 1, **dadurch gekennzeichnet, daß** man eine Verbindung der Formel II oder deren Salz 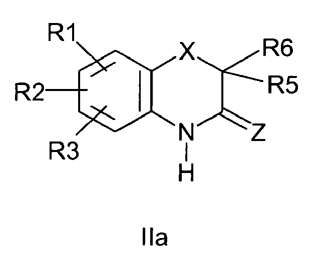 oder 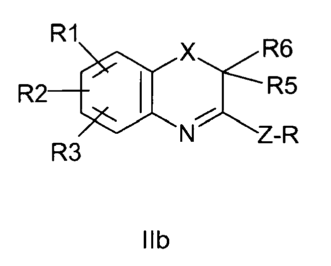 worin
R¹, R², R³, R⁵, R⁶ und X die obige Bedeutung haben, Z Sauerstoff oder Schwefel ist und R C₁₋₆-Alkyl bedeutet, mit Ammoniak oder primären Aminen umsetzt, wobei vorhandene Aminogruppen gegebenenfalls intermediär geschützt sind, und gewünschtenfalls anschließend acyliert, die Isomeren trennt oder die Salze bildet.

15. Verbindungen der Formel IIa und IIb und deren Salze 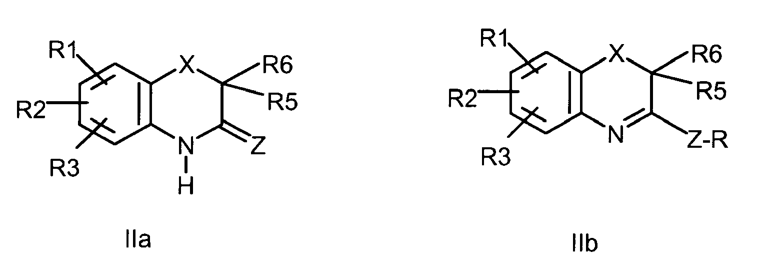 worin
R¹, R², R³, R⁵, R⁶ und X die obige Bedeutung haben, Z Sauerstoff oder Schwefel ist und R C₁₋₆-Alkyl bedeutet.

## Claims

1. Compounds of formula I, their tautomers and E- and Z-isomers, S- and R-enantiomers, diastereomers, racemates and mixtures thereof, and salts 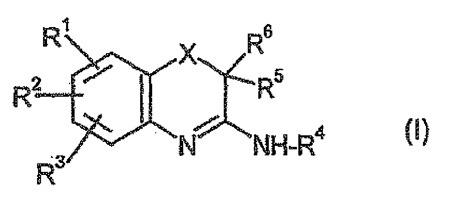 where
X is O or S,
R¹ is -(CHR⁹)ₙ-NR⁷-B,
R² is hydrogen or
R¹ and R² combine with two adjacent carbon atoms to form a 5-, 6-, 7- or 8-membered ring which is monocyclic or bicyclic, saturated or unsaturated and in which 1 or 2 CH₂ groups can be replaced by oxygen or carbonyl and which is substituted with -(CHR⁹)-NR⁷-B and can be substituted with C₁₋₄-alkyl,
R³ is hydrogen, halogen, NO₂, cyano, CF₃, -OCF₃, -S-R⁹, -O-R⁹, C ₃₋₇-cycloalkyl -NR⁹-C(=NR¹⁰)-R¹¹ -NH-CS-NR¹²R¹³, NH-CO-NR¹²R¹³, -SO₂R¹²R¹³, -CO-NR¹²R¹³, -CO-R¹⁴ NR¹⁵R¹⁶, C₆₋₁₀aryl, which is optionally substituted with halogen, cyano, C₁₋₄-alkyl, -S-R⁹ or -O-R⁹,
5- or 6-membered heteroaryl with 1-4 oxygen, sulphur or nitrogen atoms
C₁₋₆-alkyl which is optionally substituted with halogen, OR⁹, SR⁹, NR¹²R¹³, =NR¹², =NOC₁₋₆-alkyl, =N-NHaryl, phenyl, C₃₋₇-cycloalkyl or 5- or 6-membered heteroaryl,
C₂₋₆-alkenyl, which is optionally substituted with halogen, CONH₂, C≡N or phenyl,
C₂₋₆-alkynyl, which is optionally substituted with halogen, CONH₂, C≡N or phenyl,
R⁴ is hydrogen or acyl,
R⁵ and R⁶ are independently hydrogen, C₃₋₇cyclo-alkyl, phenyl, C₁₋₆-alkyl, C₂₋₆-alkenyl or C₂₋₆-alkynyl radicals, which can each be substituted with halogen, OH, O-C₁₋₆-alkyl, SH, S-C₁₋₆-alkyl, NR¹⁵R¹⁶, 5- or 6-membered heteroaryl with 1-3 nitrogen, oxygen or sulphur atoms, phenyl or C₃₋₇-cycloalkyl,
R⁷ is hydrogen, C₁₋₆-alkyl, which can be substituted with phenyl, COOC₁₋₆-alkyl or COC₁₋₆-alkyl,
R⁸ is hydrogen,
A is straight-chain or branched C₁₋₆-alkylene, straight-chain or branched C₂₋₆-alkenhylene,
B is halogenated C₁₋₈-alkyl, which can be substituted with C₆₋₁₀-aryl, in which case the aryl radical can be substituted with halogen, cyano, OH, C₁₋₄-alkoxy, C₁₋₄-alkyl, CF₃ or -OCF₃, C₃₋₈-alkynyl, which can be substituted with C₆₋₁₀-aryl, -(CH₂)ₚ-C₆₋₁₀-aryl, which is substituted with -OCF₃, or C₃₋₇-cycloalkyl, which is substituted with CF₃,
n and r are each 0, 1 to 6.
P is 0 to 6,
R⁹ and R¹⁰ are each hydrogen or C₁₋₆-alkyl,
R¹¹ is C₁₋₆-alkyl, -NH₂, -NH-CH₃, -NH-CN, optionally halogen-, C₁₋₄-alkyl- or CF₃-substituted C₆₋₁₀-aryl or optionally halogen-, C₁₋₄-alkyl- or CF₃-substituted 5- or 6-membered heteroaryl with 1 to 4 nitrogen, sulphur or oxygen atoms,
R¹² and R¹³ are each hydrogen, C₁₋₆-alkyl, optionally halogen- or C₁₋₄-alkyl-substituted phenyl, optionally halogen- or C₁₋₄-alkyl-substituted benzyl or C₃-7-cycloalkyl,
R¹⁴ is hydrogen, hydroxy, C₁₋₆-alkoxy, phenyl, optionally CO₂H-, CO₂C₁₋₆-alkyl-, hydroxy-, C₁₋₄-alkoxy-, halogen-, NR¹⁵R¹⁶-, CONR¹²R¹³- or phenyl-substituted C₁₋₆-alkyl or optionally phenyl-, cyano-, CONR¹²R¹³- or CO₂C₁₋₄-alkyl-substituted C₂₋₆-alkenyl,
R¹⁵ and R¹⁶ are each hydrogen, C₁₋₆-alkyl, optionally halogen- or C₁₋₄-alkyl-substituted phenyl or optionally halogen- or C₁₋₄-alkyl-substituted benzyl, or
R¹⁵, R¹⁶ together with the nitrogen atom form a saturated 5-, 6- or 7-membered ring which can contain a further nitrogen, oxygen or sulphur atom and can be substituted with C₁₋₄-alkyl, or an optionally halogen-substituted phenyl, benzyl or benzoyl radical.

2. Compounds according to Claim 1, wherein R⁶ is hydrogen.

3. Compounds according to Claim 1 to 2, wherein R⁵ is C₁₋₆-alkyl.

4. Compounds according to Claim 1 to 3, wherein R⁴ is hydrogen.

5. Compounds according to Claim 1 to 4, wherein R³ is hydrogen.

6. Compounds according to Claim 1, wherein A is straight-chain or branched C₁₋₆-alkylene.

7. Compounds according to any one of the Claims 1-6, viz.
(2R)-methyl-3-amino-6-(2,2,3,3,3-pentafluoropropylaminomethyl)-2H-1,4-benzoxazine dihydrochloride
(2R)-methyl-3-amino-6-(4,4,4-trifluorobutylaminomethyl)-2H-1,4-benzoxazine dihydrochloride
(2R)-methyl-3-amino-6-[4,4,4-trifluorobut-(2RS)ylaminomethyl]-2H-1,4-benzoxazine dihydrochloride
(2R)-methyl-3 -amino-6-[(3R)-fluoro-3-phenyl-prop-(2R)-yl-aminomethyl]-2H-1,4-benzoxazine dihydrochloride.

8. Pharmaceutical composition comprising a compound according to any one of the Claims 1 to 7 and one or more pharmaceutically customary excipient- or adjuvant materials.

9. Use of a compound according to any one of the Claims 1 to 7 in the manufacture of a pharmaceutical composition for treating a disease selected from the group consisting of neurodegenerative diseases, autoimmune diseases, inflammatory diseases and cardiovascular diseases.

10. Use according to Claim 9 for treating neurodegenerative diseases.

11. Use according to Claim 9 wherein the disease is selected from the group consisting of cerebral ischemia, hypoxia, multiple sclerosis, amyotrophic lateral sclerosis and comparable sclerotic diseases, Parkinson's disease, Huntington's disease, Korsakoffs disease, epilepsy, vomiting, sleep disorders, schizophrenia, depression, stress, pain, migraine, hypoglycaemia, dementia, hypotension, ARDS (adult respiratory distress syndrome), sepsis, septic shock, rheumatoid arthritis, osteoarthritis, insulin-dependent Diabetes mellitus (IDDM), inflammatory disease of the pelvis/intestine (bowel disease), meningitis, glomerulonephritis, acute and chronic liver diseases, diseases through rejection (for example allogenic heart, kidney or liver transplants), inflammatory skin diseases and psoriasis.

12. Use according to Claim 11, wherein the dementia is selected from the group consisting of Alzheimer's disease, HIV dementia and presenile dementia.

13. Compounds according to any one of the Claims 1 to 7 for use in therapy.

14. Process for production of a compound of formula I according to Claim 1, **characterized in that** a compound of formula II or its salt 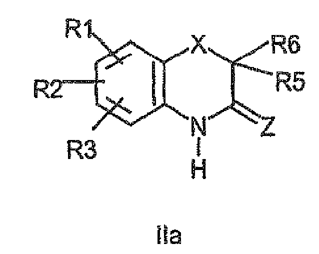 or 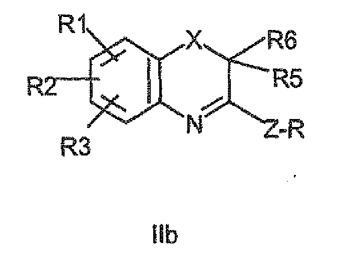 wherein
R¹, R², R³, R⁵, R⁶ and X are each as defined above, Z is oxygen or sulphur and R is C₁₋₆-alkyl, is reacted with ammonia or primary amines, wherein existing amino groups are optionally intermediately protected, and if desired subsequently acylated, the isomers are separated or the salts are formed.

15. Compounds of formula IIa or IIb and salts thereof 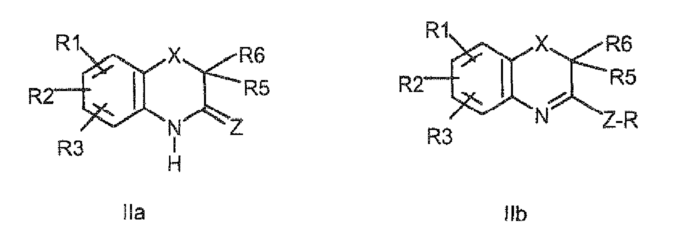 wherein
R¹, R², R³, R⁵, R⁶ and X are each as defined above, Z is oxygen or sulphur and R is C₁₋₆-alkyl.

## Revendications

1. Composés de formule I, leurs tautomères et leurs isomères E et Z, leurs énantiomères S et R, leurs diastéréoisomères, leurs racémates et leurs mélanges, et sels 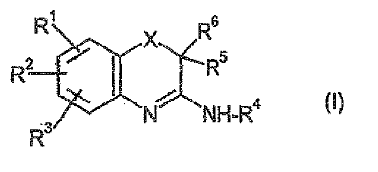 dans laquelle :
X représente un atome d'oxygène ou un atome de soufre,
R¹ représente un groupe -(CHR⁹)ₙ-NR⁷-B,
R² représente un atome d'hydrogène, ou
R¹ et R² forment, conjointement avec deux atomes de carbone voisins, un noyau à 5, 6, 7 ou 8 chaînons qui est monocyclique ou bicyclique, saturé ou insaturé, et dans lequel 1 ou 2 groupes CH₂ peuvent être remplacés par un atome d'oxygène ou un groupe carbonyle, et qui est substitué par un groupe -(CHR⁹)ᵣ-NR⁷-B et peut être substitué par un groupe alkyle en C₁₋₄,
R³ représente un atome d'hydrogène, un atome d'halogène, un groupe NO₂, un groupe cyano, un groupe CF₃, un groupe -OCF₃, un groupe -S-R⁹, un groupe -O-R⁹, un groupe cycloalkyle en C₃₋₇, un groupe -NR⁹-C(=NR¹⁰)-R¹¹, un groupe -NH-CS-NR¹²R¹³, un groupe NH-CO-NR¹²R¹³, un groupe -SO₂NR¹²R¹³, un groupe -CO-NR¹²R¹³, un groupe -CO-R¹⁴, un groupe NR¹⁵R¹⁶, un groupe aryle en C₆₋₁₀, qui est éventuellement substitué par un atome d'halogène, un groupe cyano, un groupe alkyle en C₁₋₄, un groupe -S-R⁹ ou un groupe -O-R⁹,
un groupe hétéroaryle à 5 ou 6 chaînons, renfermant de 1 à 4 atomes d'oxygène, de soufre ou d'azote,
un groupe alkyle en C₁₋₆ qui est éventuellement substitué par un atome d'halogène, un groupe OR⁹, un groupe SR⁹ un groupe NR¹²R¹³, un groupe =NR¹², un groupe =NOC₁₋₆-alkyle, un groupe =N-NH-aryle, un groupe phényle, un groupe cyclo-alkyle en C₃₋₇ ou un groupe hétéroaryle à 5 ou 6 chaînons,
un groupe alcényle en C₂₋₆ qui est éventuellement substitué par un atome d'halogène, un groupe CONH₂, un groupe C≡N ou un groupe phényle,
un groupe alcynyle en C₂₋₆ qui est éventuellement substitué par un atome d'halogène, un groupe CONH₂, un groupe C≡N ou un groupe phényle,
R⁴ représente un atome d'hydrogène ou un groupe acyle,
R⁵ et R⁶ représentent, indépendamment l'un de l'autre, un atome d'hydrogène, un groupe cycloalkyle en C₃₋₇, un groupe phényle, un groupe alkyle en C₁₋₆, des radicaux alcényle en C₂₋₆ ou alcynyle en C₂₋₆ qui peuvent être chacun substitués par un atome d'halogène, un groupe OH, un groupe O-C₁₋₆-alkyle, un groupe SH, un groupe S-C₁₋₆-alkyle, un groupe NR¹⁵R¹⁶, un groupe hétéroaryle à 5 ou 6 chaînons renfermant de 1 à 3 atomes d'azote, d'oxygène ou de soufre, un groupe phényle ou un groupe cycloalkyle en C₃₋₇,
R⁷ représente un atome d'hydrogène, un groupe alkyle en C₁₋₆ qui peut être substitué par un groupe phényle, un groupe COOC₁₋₆-alkyle ou un groupe COC₁₋₆-alkyle,
R⁸ représente un atome d'hydrogène,
A représente un groupe alkylène en C₁₋₆ linéaire ou ramifié, un groupe alcénylène en C₂₋₆ linéaire ou ramifié,
B représente un groupe alkyle en C₁₋₈ halogéné qui peut substitué par un groupe aryle en C₆₋₁₀, où le radical aryle peut être substitué par un atome d'halogène, un groupe cyano, un groupe OH, un groupe alcoxy en C₁₋₄, un groupe alkyle en C₁₋₄, un groupe CF₃ ou un groupe -OCF₃, un groupe alcynyle en C₃₋₈ qui peut être substitué par un groupe aryle en C₆₋₁₀, un groupe -(CH₂)ₚ-C₆₋₁₀-aryle qui est substitué par un groupe -OCF₃, ou un groupe cycloalkyle en C₃₋₇ qui est substitué par un groupe CF₃,
n et r valent 0, de 1 à 6,
p vaut de 0 à 6,
R⁹ et R¹⁰ représentent un atome d'hydrogène ou un groupe alkyle en C₁₋₆,
R¹¹ représente un groupe alkyle en C₁₋₆, un groupe -NH₂, un groupe -NH-CH₃, un groupe -NH-CN, un groupe aryle en C₆₋₁₀ éventuellement substitué par un atome d'halogène, un groupe alkyle en C₁₋₄ ou un groupe CF₃, ou un groupe hétéroaryle à 5 ou 6 chaînons, éventuellement substitué par un atome d'halogène, un groupe alkyle en C₁₋₄ ou un groupe CF₃, et renfermant de 1 à 4 atomes d'azote, de soufre ou d'oxygène,
R¹² et R¹³ représentent un atome d'hydrogène, un groupe alkyle en C₁₋₆, un groupe phényle éventuellement substitué par un atome d'halogène ou un groupe alkyle en C₁₋₄, un groupe benzyle éventuellement substitué par un atome d'halogène ou un groupe alkyle en C₁₋₄, ou un groupe cycloalkyle en C₃₋₇,
R¹⁴ représente un atome d'hydrogène, un groupe hydroxy, un groupe alcoxy en C₁₋₆, un groupe phényle, un groupe alkyle en C₁₋₆ éventuellement substitué par un groupe CO₂H, un groupe CO₂C₁₋₆-alkyle, un groupe hydroxy, un groupe alcoxy en C₁₋₄, un atome d'halogène, un groupe NR¹⁵R¹⁶, un groupe CONR¹²R¹³ ou un groupe phényle, ou un groupe alcényle en C₂₋₆ éventuellement substitué par un groupe phényle, un groupe cyano, un groupe CONR¹²R¹³ ou un groupe CO₂C₁₋₄-alkyle,
R¹⁵ et R¹⁶ représentent un atome d'hydrogène, un groupe alkyle en C₁₋₆, un groupe phényle éventuellement substitué par un atome d'halogène ou un groupe alkyle en C₁₋₄, ou un groupe benzyle éventuellement substitué par un atome d'halogène ou un groupe alkyle en C₁₋₄, ou
R¹⁵ et R¹⁶, conjointement avec l'atome d'azote, forment un noyau saturé à 5, 6 ou 7 chaînons, qui peut renfermer un atome d'azote, d'oxygène ou de soufre supplémentaire et peut être substitué par un radical alkyle en C₁₋₄ ou un radical phényle, benzyle ou benzoyle, éventuellement substitué par un atome d'halogène.

2. Composés selon la revendication 1, dans lesquels R⁶ représente un atome d'hydrogène.

3. Composés selon les revendications 1 à 2, dans lesquels R⁵ représente un groupe alkyle en C₁₋₆.

4. Composés selon les revendications 1 à 3, dans lesquels R⁴ représente un atome d'hydrogène.

5. Composés selon les revendications 1 à 4, dans lesquels R³ représente un atome d'hydrogène.

6. Composés selon la revendication 1, dans lesquels A représente un groupe alkylène en C₁₋₆ linéaire ou ramifié.

7. Composés selon l'une quelconque des revendications 1 à 6, à savoir :
le dichlorhydrate de (2R)-méthyl-3-amino-6-(2,2,3,3,3-pentafluoropropylaminométhyl)-2H-1,4-benzoxazine,
le dichlorhydrate de (2R)-méthyl-3-amino-6-(4,4,4-trifiuorobutylaminométhyl)-2H-1,4-benzoxazine,
le dichlorhydrate de (2R)-méthyl-3-amino-6-[4,4,4-trifluorobut-(2RS)-ylaminométhyl]-2 H-1,4-benzoxazine,
le dichlorhydrate de (2R)-méthyl-3-amino-6-[(3R)-ffuoro-3-phénylprop-(2R)-ylaminométhyl]-2H-1,4-benzoxazine.

8. Médicament comprenant un composé selon l'une quelconque des revendications 1 à 7 et un ou plusieurs supports ou auxiliaires pharmaceutique-ment habituels.

9. Utilisation d'un composé selon l'une quelconque des revendications 1 à 7 pour la production d'un médicament destiné au traitement d'une maladie qui est choisie parmi le groupe constitué de maladies neurodégénératives, de maladies auto-immunes, de maladies inflammatoires et de maladies cardio-vasculaires.

10. Utilisation selon la revendication 9 pour le traitement de maladies neurodégénératives.

11. Utilisation selon la revendication 9, dans laquelle la maladie est choisie parmi le groupe constitué d'une ischémie cérébrale, d'une hypoxie, de la sclérose en plaques, de la sclérose latérale amyotrophique et de maladies sclérotiques comparables, de la maladie de Parkinson, de la maladie de Huntington, de la maladie de Korsakoff, de l'épilepsie, de vomissements, de troubles du sommeil, de la schizophrénie, d'une dépression, du stress, d'une douleur, d'une migraine, de l'hypoglycémie, de la démence, de l'hypotension, du SDRA (syndrome de détresse respiratoire chez l'adulte), de la septicémie, d'un choc septique, de la polyarthrite rhumatoïde, de l'arthrose, du diabète sucré insulino-dépendant (IDDM), d'une maladie inflammatoire du pelvis/de l'intestin (maladie intestinale), d'une méningite, de la glomérulonéphrite, de maladies hépatiques aiguës et chroniques, de maladies dues à un rejet (par exemple, de greffes allogéniques du coeur, du foie et du rein), de maladies cutanées inflammatoires et du psoriasis.

12. Utilisation selon la revendication 11, dans laquelle la démence est choisie parmi le groupe constitué de la maladie d'Alzheimer, de la démence due au VIH et de la démence présénile.

13. Composés selon l'une quelconque des revendications 1 à 7 pour une utilisation thérapeutique.

14. Procédé de préparation d'un composé de formule I selon la revendication 1, **caractérisé en ce qu'**un composé de formule II ou son sel 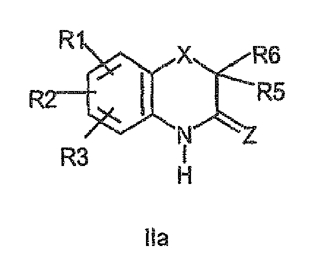 ou 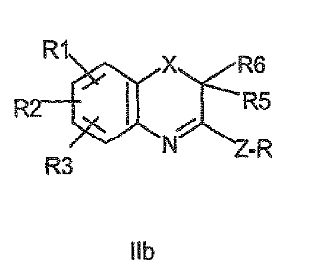 dans laquelle :
R¹, R², R³, R⁵, R⁶ et X présentent la signification ci-dessus, Z représente un atome d'oxygène ou un atome de soufre, et R¹ représente un groupe alkyle en C₁₋₆, est mis à réagir avec de l'ammoniac ou des amines primaires, les groupes amino présents étant éventuellement protégés de façon intermédiaire, et ensuite acylé, si on le souhaite, les isomères sont séparés ou les sels sont formés.

15. Composés de formule IIa ou IIb et leurs sels 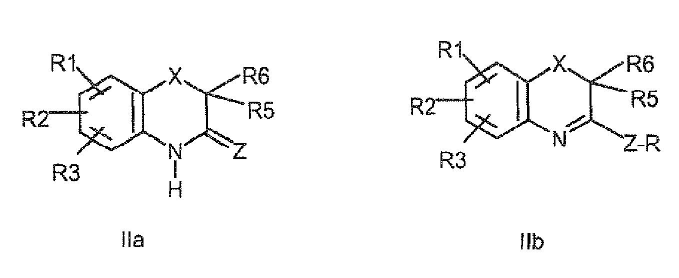 dans lesquels :
R¹ ,R² R³, R⁵, R⁶ et X présentent la signification ci-dessus, Z représente un atome d'oxygène ou un atome de soufre et R représente un groupe alkyle en C₁₋₆.
